# EUROPEAN PATENT SPECIFICATION

(11) **EP 1 896 603 B1**
(45) Date of publication and mention of the grant of the patent: **06.11.2013**
(21) Application number: 06762232.4
(22) Date of filing: 28.06.2006
(51) Int. Cl.: C12P 21/02, C07K 14/31

(54) **BIOSYNTHETIC PROCESS FOR THE PREPARATION OF GALLIDERMIN**
BIOSYNTHETISCHES VERFAHREN ZUR HERSTELLUNG VON GALLIDERMIN
PROCEDE DE BIOSYNTHESE DESTINE A LA PREPARATION DE LA GALLIDERMINE

(30) Priority: 29.06.2005 EP 05394019
(43) Date of publication of application: 12.03.2008
(73) Proprietor: Biotech Concepts GmbH, 8049 Zürich (CH)
(72) Inventor: MINAS, Wolfgang, CH-8049 Zurich (CH); PANKE, Sven, CH-8050 Zürich (CH); VALSESIA, Giorgia, CH-6512 Giubiasco (CH); MEDAGLIA, Giovanni, CH-6528 Camorino (CH)
(74) Representative: O'Brien, John Augustine
(86) International application number: PCT/EP2006/006240
(87) International publication number: WO 2007/000332

(56) References cited:
- EP-A1- 0 342 486
- DE-A1- 10 261 782
- US-A1- 2004 009 550
- F. GÖTZ , G. JUNG: "Biosynthesis of the Lantibiotics Epidermin and Gallidermin" in Microbial Fundamentals of Biotechnology" [Online] 28 January 2005 (2005-01-28), WILEY-VCH VERLAG GMBH. EDITORS: VOLKMAR BRAUN, FRIEDRICH GÖTZ , XP002417045 ISBN: 3-527-30615-3 Retrieved from the Internet: URL:http://www3.interscience.wiley.com/cgi -bin/summary/109870064/SUMMARY> Page 72, paragraph 3.6 - page 74 page 52 - page 92
- OTTENWÄLDER B ET AL: "Isolation and characterization of genetically engineered gallidermin and epidermin analogs." APPLIED AND ENVIRONMENTAL MICROBIOLOGY NOV 1995, vol. 61, no. 11, November 1995 (1995-11), pages 3894-3903, XP002415311 ISSN: 0099-2240
- KIES S ET AL: "Control of antimicrobial peptide synthesis by the agr quorum sensing system in Staphylococcus epidermidis: Activity of the lantibiotic epidermin is regulated at the level of precursor peptide processing" PEPTIDES, ELSEVIER, AMSTERDAM, US, vol. 24, no. 3, March 2003 (2003-03), pages 329-338, XP002357259 ISSN: 0196-9781

### SEQUENCE LISTING

<110> Biotech Concepts GmbH
<120> Biosynthetic process for preparation of antibiotic compounds
<130> BIOT01/C/WO
<160> 12
<170> PatentIn version 3.3
<210> 1
   <211> 14
   <212> PRT
   <213> Artificial Sequence
<220>
   <223> Truncated amino acid sequence of the pre-gallidermin leader peptide produced by a modified strain of Staphlylococcus gallinarum, Staphylococcus gallinarum DgdmP::kan having a depository number of DSM 17239
<400> 1
   Val Asn Ala Lys Glu Ser Asn Asp Ser Gly Ala Glu Pro Arg
   1               5                   10
<210> 2
   <211> 12
   <212> PRT
   <213> Artificial sequence
<220>
   <223> Truncated amino acid sequence of the pre-gallidermin leader peptide produced by a modified strain of Staphlylococcus gallinarum, Staphylococcus gallinarum DgdmP::kan having a depository number of DSM 17239
<400> 2
   Ala Lys Glu Ser Asn Asp Ser Gly Ala Glu Pro Arg
   1               5                   10
<210> 3
   <211> 30
   <212> PRT
   <213> Artificial sequence
<220>
   <223> Expected amino acid sequence of the pre-gallidermin leader peptide produced by Staphlylococcus gallinarum and a modified strain of Staphlylococcus gallinarum, Staphylococcus gallinarum DgdmP::kan having a depository number of DSM 17239
<400> 3
   Met Glu Ala Val Lys Glu Lys Asn Glu Leu Phe Asp Leu Asp Val Lys
   1               5                   10                  15
   Val Asn Ala Lys Glu Ser Asn Asp Ser Gly Ala Glu Pro Arg
<210> 4
   <211> 32
   <212> DNA
   <213> artificial sequence
<220>
   <223> gdmP-PstI-F forward primer derived from an internal fragment of the Staphylococcus gallinarum gdmP gene
<400> 4
   catatctgca gggtttgtag cgcatcataa tc 32
<210> 5
   <211> 34
   <212> DNA
   <213> artificial sequence
<220>
   <223> gdmP-HindIII-R reverse primer derived from an internal fragment of the Staphylococcus gallinarum gdmP gene
<400> 5
   cggtcacaag cttagtaagt cccaagtaga gtcc 34
<210> 6
   <211> 52
   <212> DNA
   <213> artificial sequence
<220>
   <223> kan-Rreverse primer derived from Bacillus subtilus kanamycin resistance gene
<400> 6
   cctacaatat taatagcaat catattattt cccttcaaaa caattcatcc ag 52
<210> 7
   <211> 30
   <212> DNA
   <213> artificial sequence
<220>
   <223> gdmQ-EcoRI-2-F forward primer derived from an internal fragment of the Staphylococcus gallinarum gdmP gene
<400> 7
   cggaattcgt ctatcaattc atcatcaatg 30
<210> 8
   <211> 39
   <212> DNA
   <213> artificial sequence
<220>
   <223> gdmQ-R reverse primer derived from the 3' end of the Staphylococcus gallinarum gdmP gene
<400> 8
   tgaagggaaa taatatgatt gctattaata ttgtaggtg 39
<210> 9
   <211> 27
   <212> DNA
   <213> artificial sequence
<220>
   <223> kan-ClaI-F forward primer derived from Bacillus subtilus kanamycin resistance gene
<400> 9
   ttatcgatgc cgtatgtaag gattcag 27
<210> 10
   <211> 25
   <212> DNA
   <213> artificial sequence
<220>
   <223> PROOF-K forward primer derived from an internal fragment of the B.subtilus kanamycin resistance gene
<400> 10
   acgaactcca attcactgtt ccttg 25
<210> 11
   <211> 26
   <212> DNA
   <213> artificial sequence
<220>
   <223> PROOF-P reverse primer derived from an internal fragment of the Staphylococcus gallinarum gdmP gene
<400> 11
   ggtgaggggt gctatatgaa gaaatt 26
<210> 12
   <211> 30
   <212> DNA
   <213> artificial sequence
<220>
   <223> PROOF-Q forward primer derived from an internal fragment of the Staphylococcus gallinarum gdmQ gene
<400> 12
   ctttgcacac ccttaaatta tctcttaatc 30

## Description

The invention relates to a process for the biosynthesis and production of gallidermin, a modified strain for producing an in-active pre-form of gallidermin and, to the use thereof.

### Background of the Invention

Many Gram-positive bacteria produce peptide antibiotics called lantibiotics (lanthionine-containing antibiotics) which contain lanthionine, an unusual aminoacid added to the precursor peptide post-translationally (Jack et al., 1998). Gram-negative bacteria also produce similar molecules, bacteriocins, but these are larger proteins that act on target receptors and have a narrow spectrum of activity.

Lantibiotics are small (20 - 40 amino-acids) cationic peptides whose synthesis starts with a ribosomally synthesized pre-pro-protein, which undergoes posttranslational modification to yield the lanthionine containing but still inactive pre-form of the antibiotic, which is then exported from the cell and either during or after export activated by proteolytic cleavage by lantibiotic specific serine-protease. The producer strains possess so called immunity functions that protect to some extent against the action of the lantibiotic but which does not confer resistance (Jack et al., 1998; Hille et al., 2001). Lantibiotics are expressed during exponential and into early-stationary growth-phases meaning that they reduce competition from other flora once they themselves have become established.

Several classes of lantibiotics have been described that group the molecules according to their structure in solution (Jack et al., 1998). Type-A lantibiotics, damage the bacterial cells by disruption of anionic membranes to form wedgelike pores driven by voltage differences across membranes. This allows efflux of metabolites, ions and solutes from the bacterial cell. In addition, type-A lantibiotics bind to Lipid II, an essential precursor (carrier) for membrane building blocks. Depending on the size of the lantibiotic, two modes of killing are currently discussed (Bonelli et al. 2006, Breukink & Kruijff 2006).

Members of the typ-A lantibiotic group include cytolysin L1 and L2 from *Enterococcus faecalis*, epidermin, epilancins and Pep5 from *Staphylococcus epidermidis*, gallidermin from *Staphylococcus gallinarum,* lacticin 481, mutacins from *Streptococcus mutans*, nisin A and Z from *Lactococcus lactis*, salavaricin A from *Streptococcus salivarius*, subtilin from *Bacillus subtilis*, and variacin from *Micrococcus varians.*

Despite of being of clinical value no type-A lantibiotic have been developed into a clinical products, even though several have proven their potential to treat peptic ulcers (nisin, *H. pylori, Clostridium difficile*; CA2291645), MRSA protection in mice (mersacidine, *B. subtilis*) and others (Jack et al.,1998). Lysostaphin is being developed as a wound-anti-infective agent and in the treatment of *Staphylococcus aureus* infections. Mucatcins (*Streptococcus mutans*) are being evaluated for mouth wash and tooth paste applications. Nisin is currently the only marketed product used for food preservation (de Vuyst & Vandamme, 1993; US 4,584,199; US 4,597,972; US 4,716,115).

Gallidermin is a molecule of particular interest as it possibly provides a treatment for acne or wound infection or endocarditis in human or for bovine mastitis (gallidermin, S. *gallinarum*; US 5,710,124),

The major obstacle to an industrial scale production of pharmaceutical grade gallidermin or any lantibiotic is the toxicity of the lantibiotic molecules to the producing strain, resulting in the very low product titres in the fermentation broth and in the rather complex experimental processes with on-line product recovery (Kempf et al., 2001). Also feed-back inhibition by the product can prevent high level expression.

Any process to overcome this limitation in production of gallidermin would have valuable therapeutic potential enabling the economic production and further development of this compound or similar compounds in to therapeutic drugs that are urgently needed to combat the growing threat of infections by multidrug-resistant pathogenic bacteria.

### Statements of Invention

According to the invention there is provided a process for the preparation of an inactive pre-form of gallidermin comprising the steps of :-
culturing a biologically pure organism capable of producing gallidermin wherein the gallidermin specific protease (GdmP) gene of the biologically pure organism is inactivated by the insertion of a constitutive promoter cassette; and
isolating the inactive pre-form of the gallidermin from the cultivation.

In one embodiment of the invention the organism is cultivated in an aqueous cultivation medium containing assimilable sources of carbon, nitrogen and inorganic substances until substantial growth and metabolic activity is detectable.

In one embodiment of the invention the inactive pre-form of gallidermin is isolated by recovery from the cultivation medium by first separation of the biomass by centrifugation or filtration followed by a hydrophobic interaction chromatography.

In one embodiment of the invention the organism is selected from any one or more of *Staphylococcus gallinarium*, or any recombinant microbial organism expressing the genes for the production and secretion of pre-gallidermin or any pre- gallidermin or derivative thereof.

In another embodiment of the invention the inactive pre-form of gallidermin is biocatalytically activated to yield a mature and active form of gallidermin.

In one embodiment of the invention the inactive pre-form of gallidermin is biocatalytically activated by a gallidermin specific protease. The gallidermin specific protease may be selected from any one or more of ArgC, bromelain and trypsin.

In one embodiment of the invention the active form of gallidermin is separated from the protease and the cleaved off leader and unprocessed pregallidermin by chromatography.

In one embodiment of the invention the isolated preform comprises a truncated amino acid sequence of VNAKESNDSGAEPR (SEQ ID No. 1).

In another embodiment of the invention the isolated preform comprises a truncated amino acid sequence AKESNDSGAEPR (SEQ ID No. 2) or any other N-terminal truncated pre-forms.

The invention also provides a genetically modified strain of *Staphlylococcus gallinarum* wherein the gallidermin specific serine protease, (GdmP) gene is inactivated by the insertion of a constitutive promoter cassette.

The invention further provides modified strain of *Staphlylococcus gallinarum*, *Staphylococcus gallinarum* Δ*gdm*P::*kan* deposited with the Deutsche Sammlung von Microorganismen having a depository number of DSM 17239.

The invention also provides an isolated inactive pre-form of gallidermin and isolated active gallidermin as prepared by a process of the invention.

### Brief Description of the Drawings

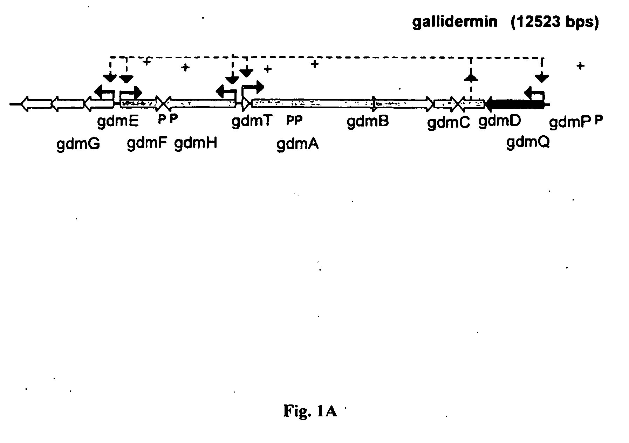

The invention will be more clearly understood from the following description of some embodiments thereof, given by way of example only, with reference to the accompanying drawings, in which:-
Fig. 1A is a schematic representation showing the genetic organization of the gallidermin biosynthetic gene cluster. Positive regulation by GdmQ is indicated by the dotted line. The directionality of promoters, marked P is indicated by arrows. Genes are marked as follows: essential for prelantibiotic synthesis (shaded box), for immunity open boxes, and the protease (solid box);

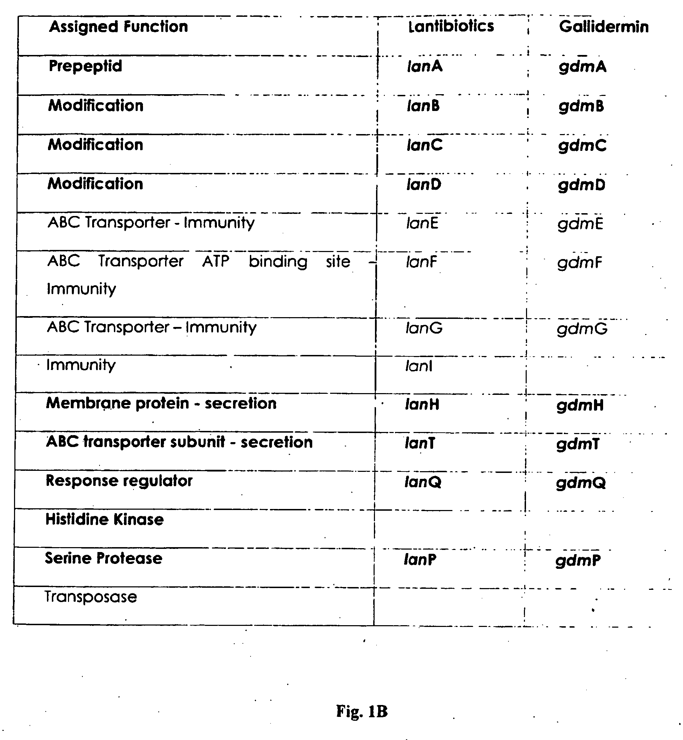

Fig. 1B is a table listing the respective genes with their assigned functions. The description *lan* is used as abbreviation for homologous genes found in several lantibiotic gene clusters. Genes essential for lantibiotic biosynthesis are marked in bold;

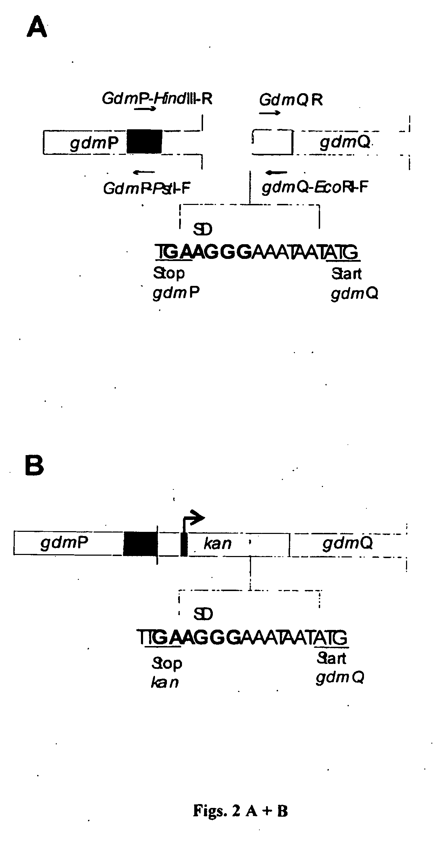

Figs. 2A and 2B are schematic representations, Fig. 2A shows the overlap of the *gdm*P stop codon with the Shine-Delgarno (SD) sequence of *gdm*Q. Small arrows indicate the sites for PCR primer annealing. Fig. 2B shows the fusion between the kanamycin-resistance cassettes stop codon with the SD sequence for *gdm*Q. The arrow indicates the newly inserted constitutive promoter, conferring kanamycin resistance and driving *gdm*Q expression;

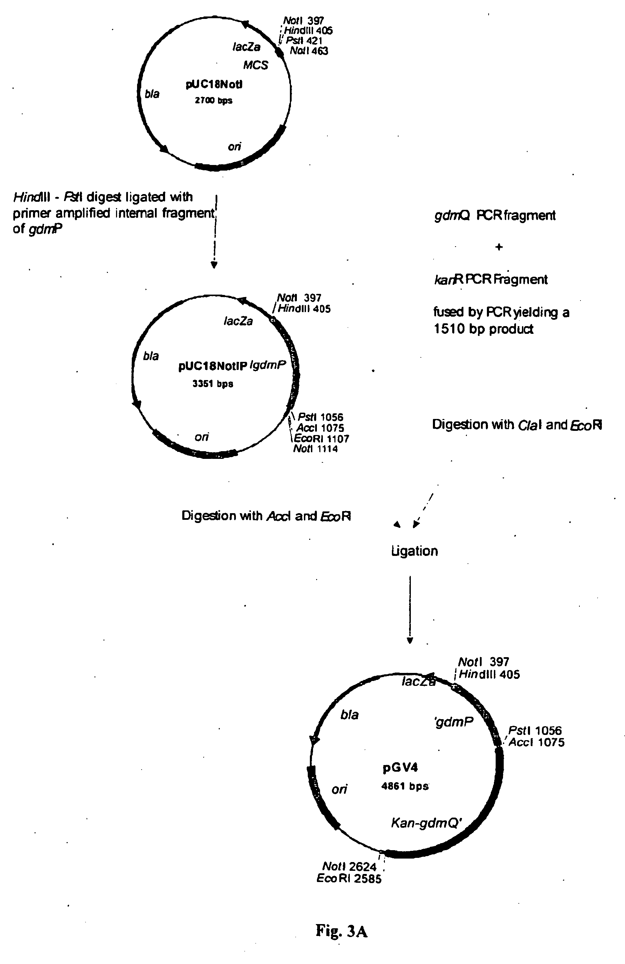

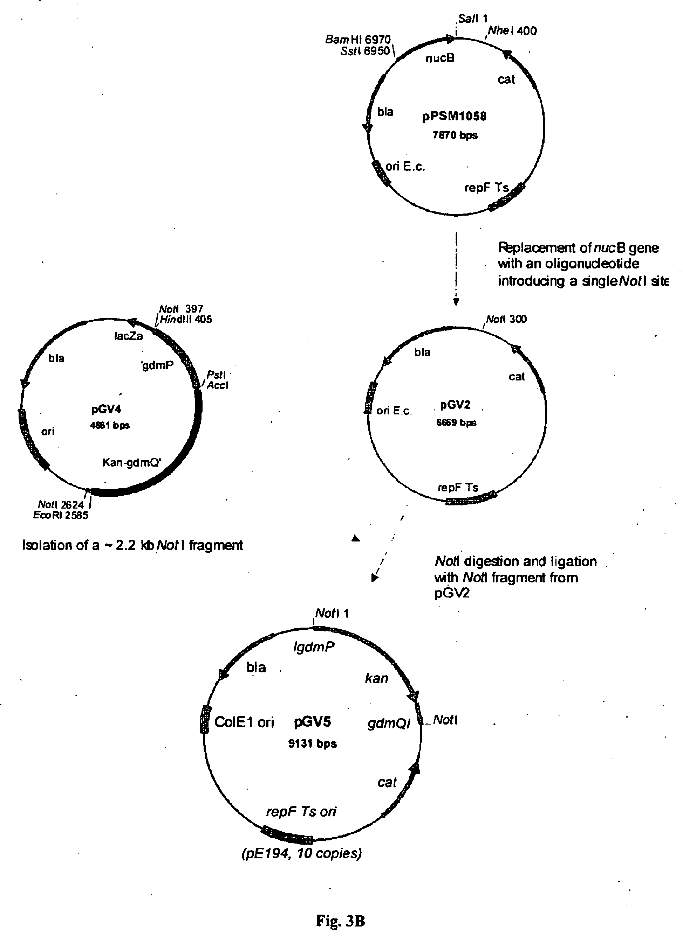

Figs. 3A and 3B show the construction of pGV5, the knock-out vector used to eliminate GdmP. Fig. 3A shows the construction of the knock-out cassette; Fig. 3B shows the path to the final knock-out shuttle vector. Relevant restriction sites and features are shown;

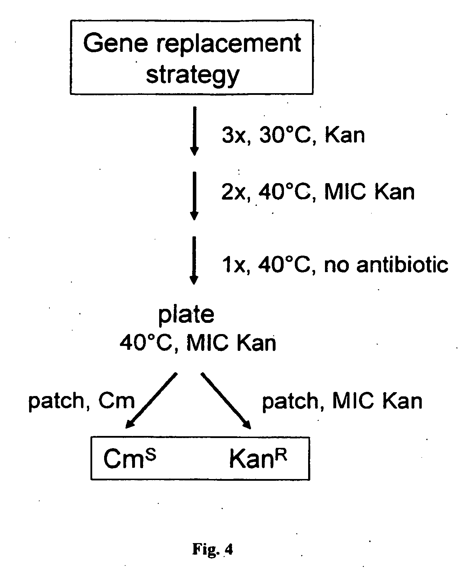

Fig. 4 is a schematic representation of the strategy employed for the selection of *gdm*P knock-out strains. Cm: chloramphenicol; Kan: kanamycin; MIC: minimum inhibitory concentration; R: resistance; S: sensitivity;

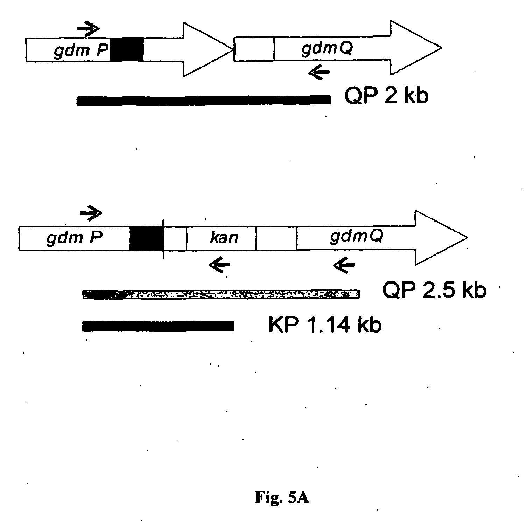

Fig. 5A is a schematic representation showing the results of the genetic analysis of wild-type and the Δ*gdm*P strain using specific primer pairs to verify the gene replacement and the proper insertion of the kanamycin-resistance cassette. The expected sizes for the PCR amplicons are shown;

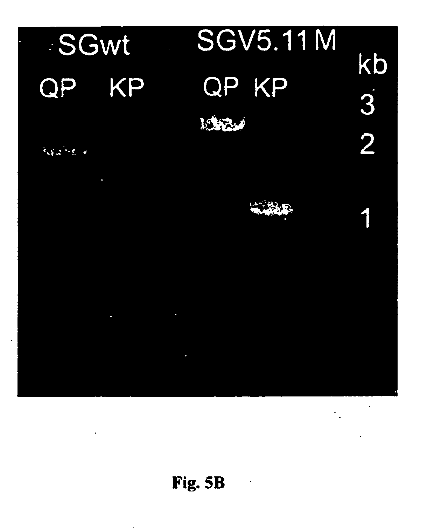

Fig. 5B shows the result of the analysis. SGwt=wild-type; SGV5.11= Δ*gdm*P; M: marker; QP:PCR reaction with primer pair PROOF-Q and PROOF-P; KP: PCR reaction with primer pair PROOF-K and PROOF-P;

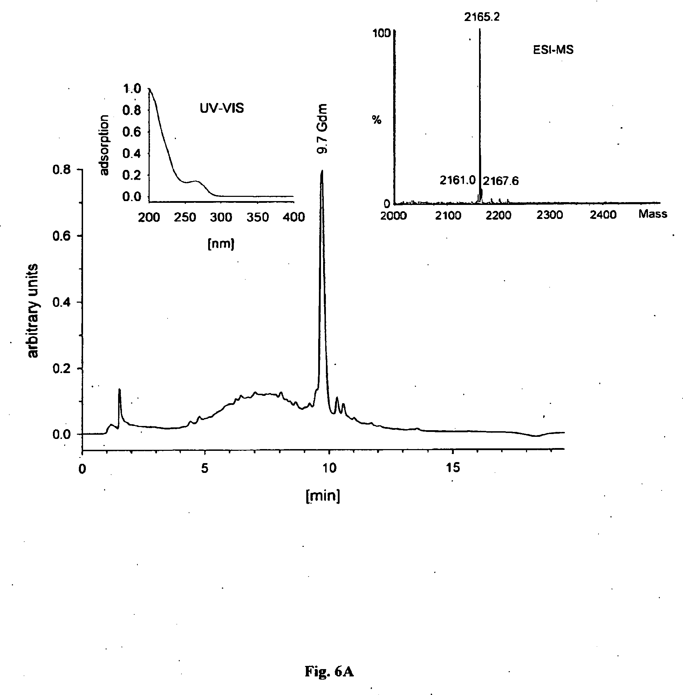

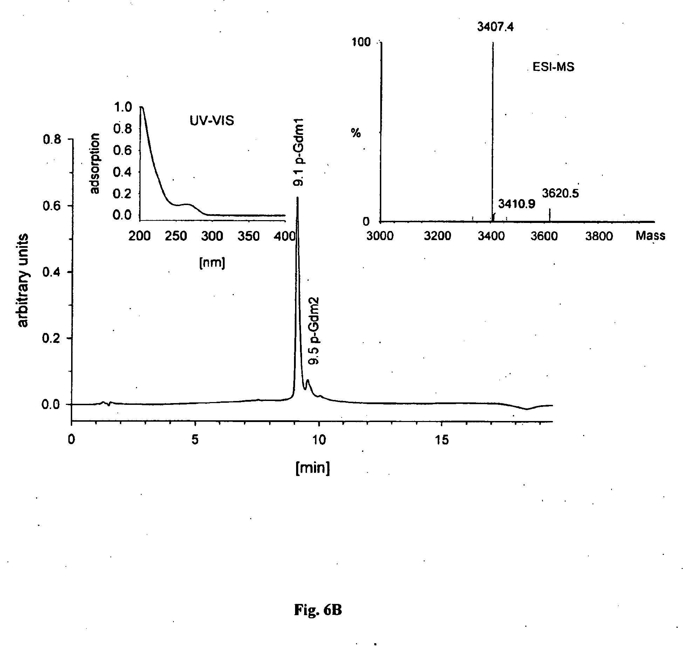

Figs. 6A and 6B are graphs of HPLC analysis of gallidermin and pregallidermin. Fig. 6A shows the HPLC analysis of gallidermin. Elution profile, UV-VIS spectra and ESI-MS spectra are inserted. The mass of 2165.2 Da corresponds to the calculated mass of 2165.5Da; gallidermin eluted at ∼9.7 minutes. Fig. 6B shows the HPLC analysis of the pregallidermins. Two main pre-gallidermins were detected eluting at ∼9.10 (p-Gdm1) and 9.5 (p-Gdm2) minutes, respectively. The ESI-MS spectrum of the major pre-gallidermin (9.1 min) is shown;

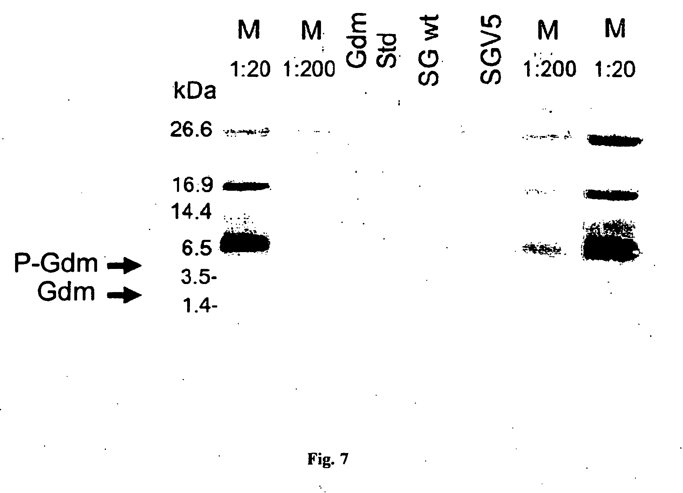

Fig. 7 is a tricine-SDS-PAGE of partially purified culture supernatants from *Staphylococcus gallinarum* wild-type (SGwt) and Δ*gdm*P::*kan* mutant strain (SGV5). Gallidermin standard (Gdm Std) was purified gallidermin used as control. After separation was completed, the gel was blotted onto nitrocellulse membrane and silver stained. (M: marker in 1:20 and 1:200 dilution). Pre-gallidermin has an expected molecular mass of 6050 Da, but only a product of about 3500 Da was detected that corresponded with the ESI-MS data in Fig. 6B. Gallidermin with of 2164 Da was detected in the wild type only;

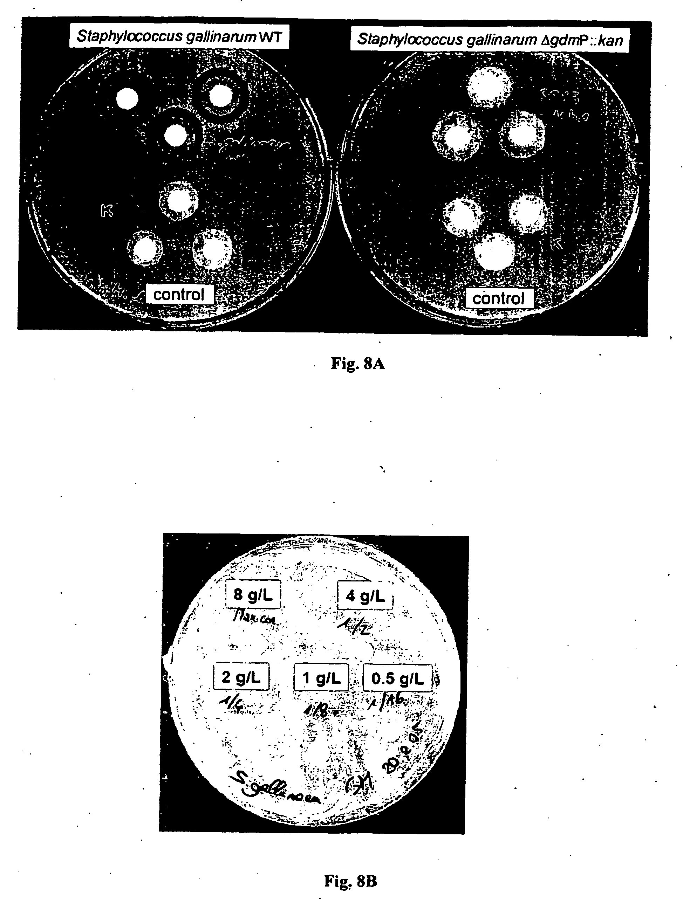

Figs. 8A and 8B show culture results. Fig. 8A shows the bioassay results from culture supernatants from wild-type *Staphylococcus gallinarum* (wt) and *Staphylococcus gallinarum* Δ*gdm*P::*kan* (SGV5); K: fresh medium Fig. 8B shows the non-toxic properties of the gallidermin precursor molecules isolates from the culture supernatant of *Staphylococcus gallinarum* Δ*gdm*P::*kan.* Numbers indicate the concentration of the solution of which 20 µl were applied onto test discs;

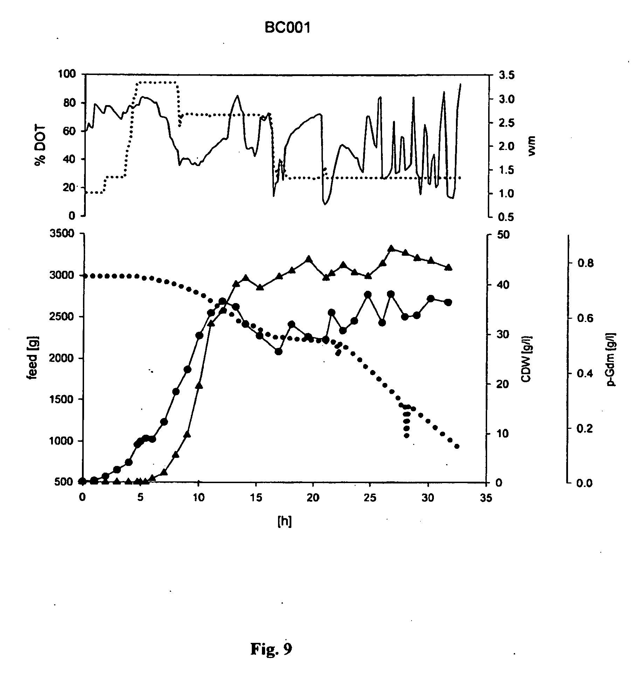

Fig. 9 is a graph showing the fermentation of *Staphylococcus gallinarum* Δ*gdm*P;;*kan* strain, the biomass formation (CDW, filled circles), pre-gallidermin formations (p-Gdm, filled triangles), maltose feed (feed, dotted line), dissolved oxygen tension (DOT) and aeration (vvm, dotted line);

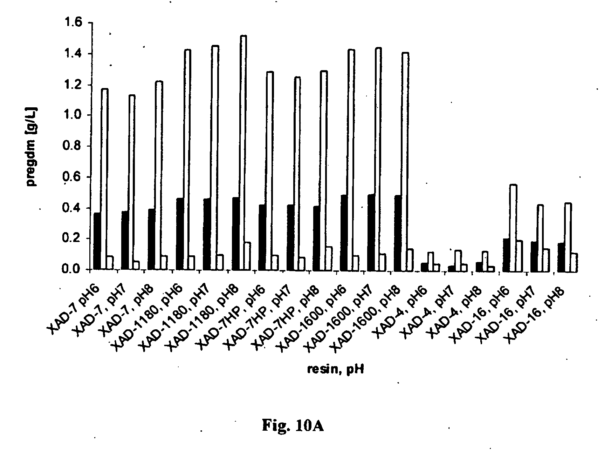

Figs. 10A to 10C Summarize the adsorption and elution properties of different Amberlite^{®} resins carried out at different pH. A: adsorption capacity (solid bars) of 80 mg (dry weight based) washed resin and recovery in two step elutions (open and gray bars); B step yield (solid bars) and recovery of adsorbed material (open bars) obtained with the different resins at various pH values. C: HPLC purity of pre-gallidermin in the eluted faction; purity was defined as HPLC area pre-gallidermin/total HPLC area.

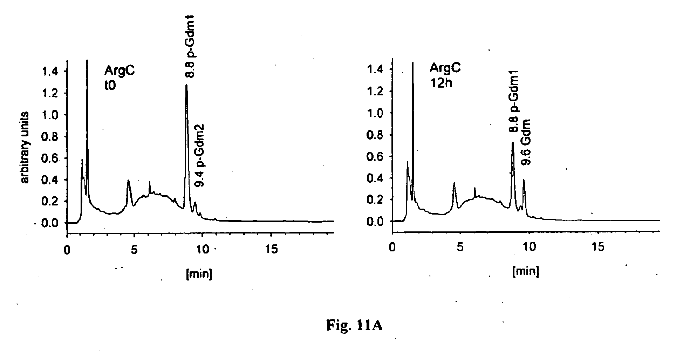

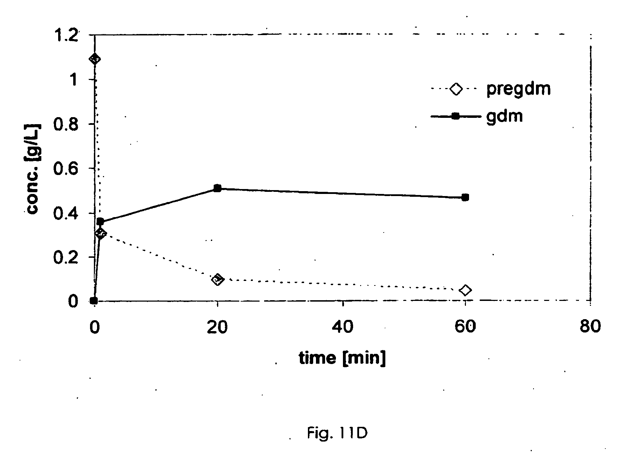

Figs. 11A to 11D are graphs showing the results of the proteolytic conversion of pre-gallidermin to gallidermin using endoproteinase ArgC (A), bromelain (B) and trypsin (C) under various conditions (see text for details). Fig. 11D shows the conversion of partially purified pregallidermin, 90% by HPCL area, obtained at pH 6.5, 20°C using 1.1g/l pre-gallidermin and 0.2 g/l trypsin.

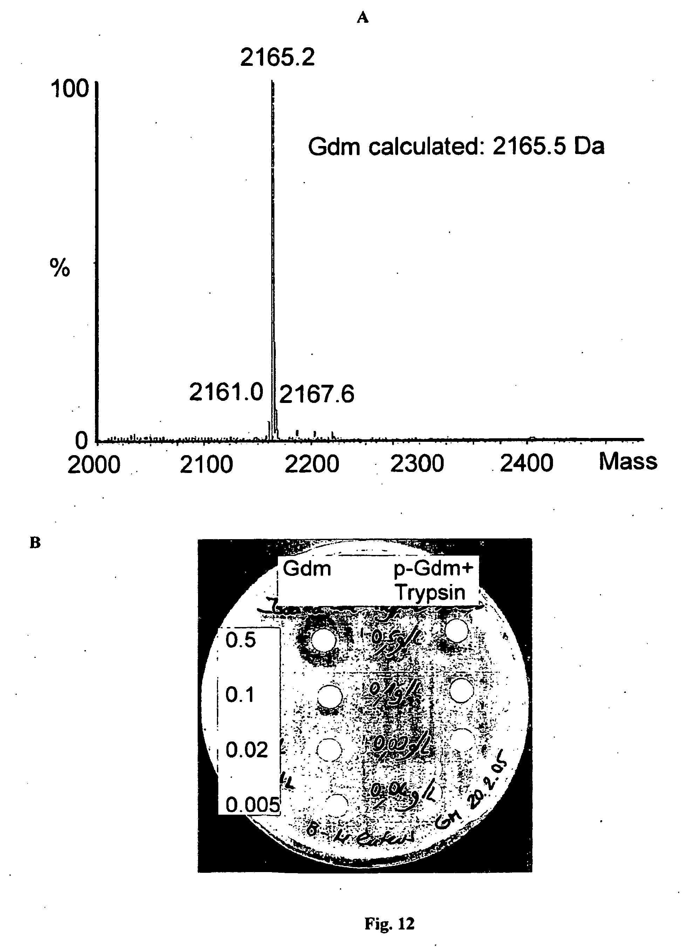

Figs. 12A and B show the result of the proteolytic (trypsin) conversion of pregallidermins into gallidermin. The ESI-MS (Fig. 12A) confirms the proper mass and processing of the premolecules to gallidermin; Fig. 12B shows the evaluation of the antibacterial activity of gallidermin isolated from the wild-type culture supernatants (left site) and in vitro processed (trypsin) from *Staphylococcus gallinarum* Δ*gdm*P;;*kan* culture supernatant (right side). Dilutions for a stock solution of either compound were applied onto test discs and incubated in the presence of a sensitive test strain. Both compounds applied at the same concentrations showed similar activities. Pre-gallidermins (1.09g/l) were incubated with trypsin (0.2g/l) 1h, RT, pH 6.5,

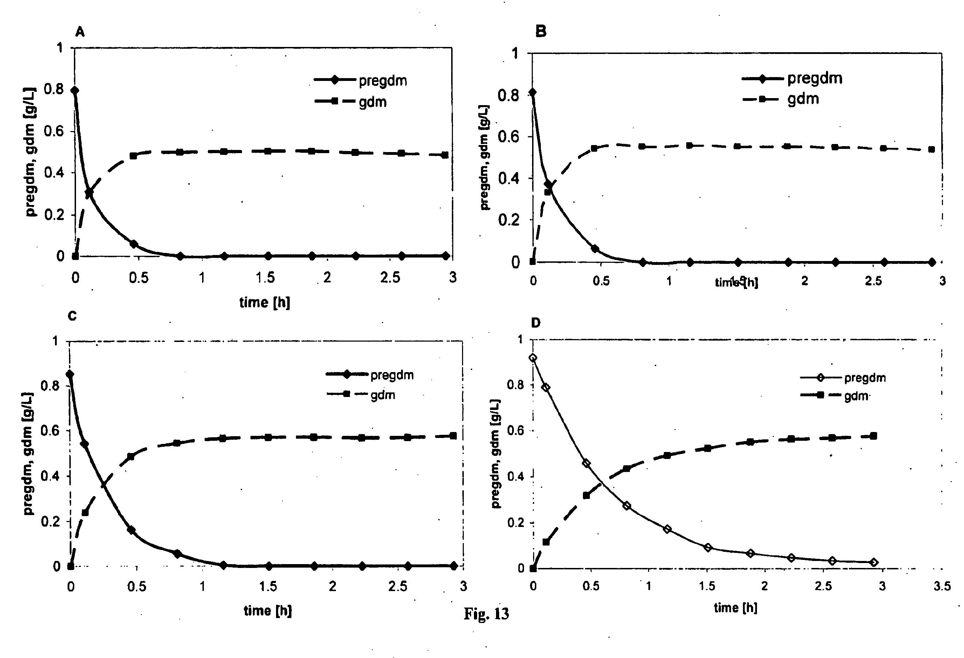

Figs. 13A to 13D show the results of tryptic cleavage of pre-gallidermin to gallidermin in the presence of different methanol concentrations. A: activation in aqueous system (100 % H₂O); B: in the presence of 20%; C 40 % and D 60% methanol; gallidermin dashed line; pre-galligermin solid line.

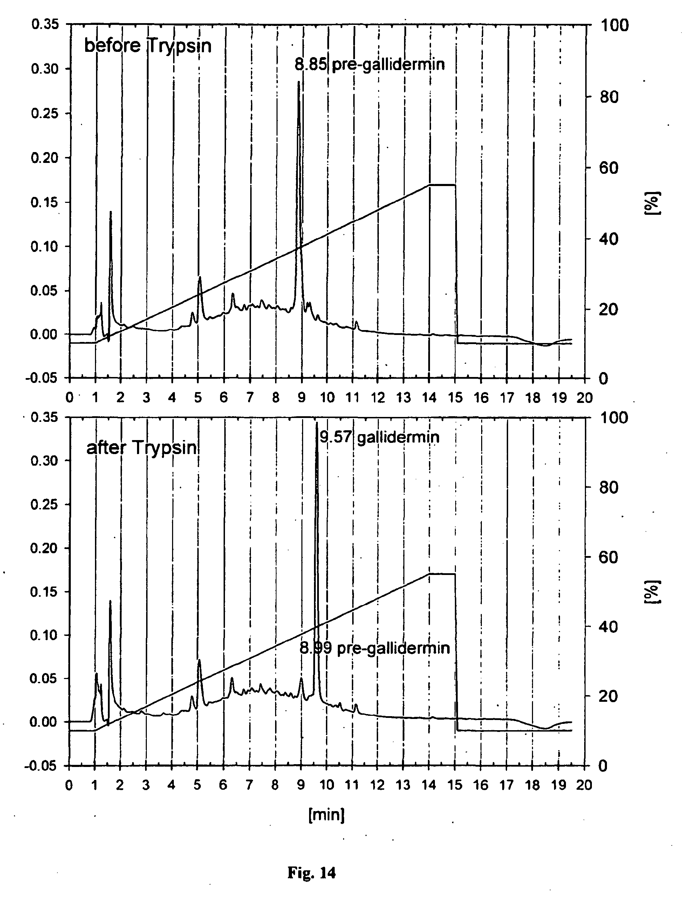

Figs 14A and B show chromatographic analysis of the pre-gallidermin to gallidermin conversion in the presence of 20% methanol at time 0 min and after 3 hours.

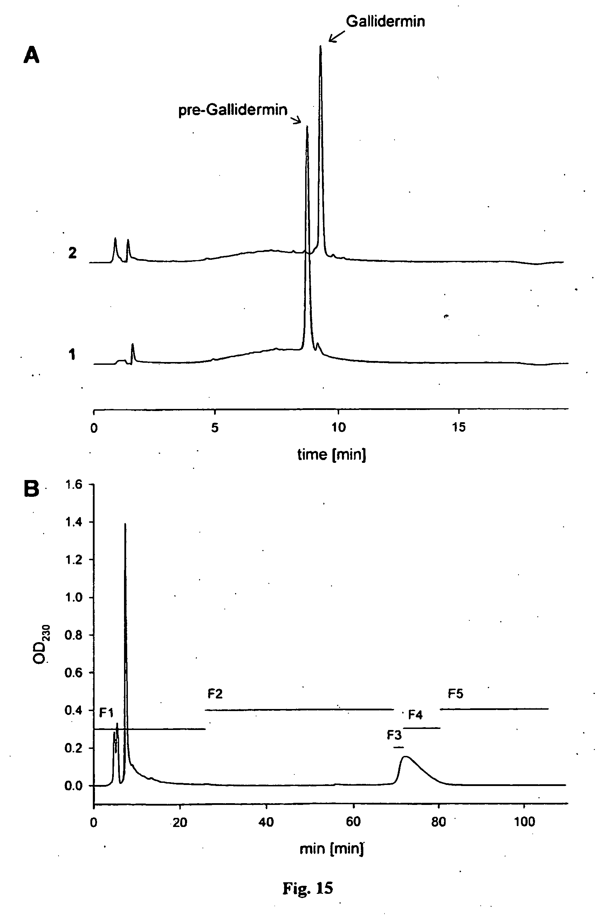

Figs 15A to C show the chromatographic analysis of partially purified gallidermin from the tryptic digest. A: conversion of pre-gallidermin (1) into gallidermin (2); B: preparative HPLC separation of maturated gallidermin from leader and trypsin with fractions collected as indicated on the chromatogram. C: Fractions MALDI-MS analysis of the fractions; F0, starting material after digest and F1 to F5 from chromatographic separation as indicated in 15 B. Arrows indicate the peaks representing gallidermin (2165.9 Da) and the cleaved off leader peptide (1260.6 Da).

### Detailed Description

We have found a new and efficient process for the production of the type-A lantibiotic gallidermin. Due to the product toxicity, including toxicity to the producing bacterium, gallidermin currently lacks a cost efficient high yielding production process for drug grade material. The process of the invention comprises a fermentative production of the respective biologically in-active and non-toxic pre-forms of gallidermin by either genetically engineered producer strains or mutants of the natural producer strains that are deficient for the extracelluar gallidermin pathway specific serine-protease. Alternatively, fermentation conditions may be selected that specifically inhibit the serine protease. The process comprises a downstream process in which the pre-form gallidermin is isolated from the culture medium and subsequently biocatalytically activated. Following this activation the mature and active gallidermin is isoloated.

The pre-gallidermin is isolated from the fermentation medium and a bio-catalytic step is carried out using commercially available proteases to process the pre-gallidermin into the mature and active gallidermin. The mature gallidermin may then be purified to yield a dry preparation.

The process provides a spatially separated production process for the preparation of active gallidermin. Throughout the following examples a native inactive pre-form of gallidermin is made by a genetically modified microbe, alternatively, an engineered inactive pre-form of gallidermin may be made, that is isolated and then in vitro activated by simple enzymatic cleavage.

Bacterial hosts and fermentation processes are described that predominantly yield pre-forms of gallidermin. A process for the purification of the pre-forms of gallidermin is described. Also processes are described using specific serine proteases that have the desired specificity to proteolytically cleave the pre-forms of gallidermin into their respective mature and active form. Purification of the activated gallidermin is also described.

A genetically modified strain of special interest is *Staphylococcus gallinarum* BC001 which has been deposited with the Deutsche Sammlung von Microorganismen, Mascheroder Weg 1B, 38124 Braunschweig, Germany under the terms of the Budapest Treaty on 13. April 2005 having a depository number of DSM 17239.

Gallidermin also binds to the membrane lipid II inhibiting the cell wall biosynthesis andinducing autolysis of the cells. Interestingly an in contrast to other, larger type-A lantibiotics, gallidermin is too short to span the membrane and to induce pores nevertheless, it acts on growing and non-growing cells very effectively (Bonelli et al., 2006; Breukink & Kruijff, 2006; Sahl & Bierbaum, 1998).

Gallidermin also inhibits cell wall biosynthesis by binding at the cytoplamic precursor, Lipid I, which would imply a membrane crossing of gallidermin. In contrast to vancomycin that recognizes the D-ala-D-alanyl residues of the peptide subunit of the peptidoglycan, gallidermin binds to the disaccharide moiety of the glycan chain that has been studied in details for nisin, a molecule that shares the respective features, a pyrophosphate cage structure, with gallidermin (Breukink & Kruijff, 2006). Gram-negative cells can also be sensitized by disrupting the outer membrane integrity (with e.g. EDTA *in vitro*)*.* Furthermore, as the producing organism does not possess a lantibiotic resistance gene (and in the new process will not be forced to develop one, neither is one introduced), we can ensure that any gallidermin preparation will be free of any DNA from which infectious bacteria could acquire lantibiotic resistance.

Despite the chemical similarities among the type-A lantibiotics, distinct differences in their mode of action and their primary targets have been recognized that are also reflected in different spectrum of sensitive bacteria and by different minimal inhibitory concentrations (MIC) needed to exhibit their antibiotic activity (US 5,231,013; Kellner et al., 1988).

Mammalian cells are spared because they contain cholesterol which is less anionic.

The genetics and the biosynthesis of these lantibiotics has been described in numerous publications (for review: Jack et al., 1998; Sahl & Bierbaum, 1998; McAuliffe, et al. 2001). In particular, gallidermin has been described in detail in US5,231,013. In general, a ribosomally produced pre-pro-peptide of the antibiotic is synthesized. This in-active pre-pro-peptide is comprised of a signal of leader peptide needed for export and the polypeptide chain that does not yet contain any dehydroamino residues and/or thioester bridges, typical for lantibiotics. The first processing of a pre-sequence polypeptide is carried out by an enzymatic complex which effects formation of dehydroamino residues and/or thioether bridges. This modified but still in-active so called pre-peptide is then exported by a dedicated lantibiotic transporter and further modified by proteolytic cleavage of the leader peptide. Both export and proteolytic functionality are encoded within the biosynthetic gene cluster. Since the active molecule exhibits toxicity to the producing organism, immunity or tolerance against these compounds is achieved by so called immunity genes found in the biosynthetic gene cluster as well. Nevertheless, immunity, the energy dependent clearance of the membrane from lantibiotics is limited to low levels, hence limiting biosynthesis and production of the lantibiotic. All genes encoding the enzymes required for these functions are encoded within the respective biosynthetic gene clusters (Fig. 1A/B). Increased tolerance to lantibiotics may also derive from changes in the membrane composition that reduce the membrane fluidity.

The major drawback for the clinical development and commercialisation of gallidermin are the high costs of production due to low fermentation titres, a result of product toxicity for the producing bacterium and in some cases negative feed-back regulation of the biosynthesis. A second problem is the low recovery during downstream processing, which is at least in part a consequence of the earlier process.

We have found a process for overcoming the low productivity of the system by eliminating the auto-toxicity of gallidermin. As a result an economic production process for synthesising gallidermin can be achieved.

The process involves fermentatively producing pre-forms of gallidermin, such as pre-gallidermin, the immediate precursor of gallidermin, rather than the mature gallidermin itself. The pre-gallidermin is essentially non-toxic to the producing strain, and since biosynthesis of the lantibiotics is growth associated, overcoming the toxicity effect leads to an increased gallidermin production. This also provides the basis for further improving strain productivity by genetic and metabolic engineering. Following the isolation and purification of the pre-gallidermin from the culture broth, the active gallidermin is obtained in an *in vitro* biocatalytic step. This activation is connected to a radical change in the molecular properties compared to the fermentatively produced compound that is utilized for further downstream processing (DSP) for preparing the purified gallidermin.

The invention will be more clearly understood by the following examples. The examples presented are illustrative only and various changes and modifications within the scope of the invention will be apparent to those skilled in the art.

### Example 1

### Construction of a pre-gallidermin producing strain

The natural producer of gallidermin is *Staphylococcus gallinarum* Tü3928. This strain carries in its chromosome all nine genes (14 kb) required for the biosynthesis and its regulation, postranslational modification, export, immunity and a gallidermin specific serine protease, designated GdmP (Fig. 1A/B; Götz & Jung, 2001; Hille, 2002). For pre-gallidermin production, said GdmP serine-type protease needs to be inactivated. Several genetic approaches for inactivating GdmP are possible to those skilled in the art, including an in frame deletion of the gene, insertional disruption and inactivation, or by preventing m-RNA translation, e.g. using antisense sequences. Alternatively, the *gdm*P product, the protease, can be chemically inactivated during fermentation.

Here the inactivation of the *gdm*P gene is carried out by insertional disruption. Care has to be taken for the expression of the translationally coupled, downstream located *gdm*Q gene, which is essential for regulation of gallidermin production. Preceding *gdm*P is an *E. coli*-like -10 region (5'-TATAAA) 12 bp in front of the Shine-Delgarno (SD) sequence which may serve as a promoter in staphylococci. The distance between the *gdm*P stop codon and the ATG start codon of *gdm*Q is only 10 nucleotides and the *gdm*Q SD sequence overlaps with the *gdm*P termination codon as shown in Fig. 2A. Therefore, the constitutively expressed promoter from the *Bacillus subtilis* kanamycin-resistance gene (*kan*^{r}), isolated from plasmid pDG782 (Guerot-Fleury et al., 1995), was inserted upstream of *gdm*Q Fig. 2B. Standard methods well known in the art are applied in the preparation of, amplification, sequencing and cloning of DNA. (preferred general methods are described in Sambrook et al. (supra)).

First an internal *gdm*P fragment from *S. gallinarum* was amplified by PCR, using the primer pair *gdm*P-*Pst*I-F (CATATCTGCAGGGTTTGTAGCGCATCATAA TC) (SEQ ID No. 4) and *gdm*P-*Hind*III-R (CGGTCACAAGCTTAGTAAGTC CCAAGTAGAGTCC) (SEQ ID No. 5). PCR amplification was performed with an annealing temperature of 58°C, and produced a 651 bp-long amplicon. This was double digested with *Pst*I and *Hind* III, and then cloned into pUC18NotI (Herrero et al., 1990). The resulting plasmid was named pUC18NotI-P (Fig. 3A).

Since *gdm*Q had no functional promoter, the gene was placed under control of the *kan*^{R} promoter, a *kan*^{R}*-gdm*Q fusion was created by overlap extension PCR fusing the kanamycin resistance gene without its termination sequence to the 364 bp *gdm*Q fragment. The primer *kan*-R (CCTACAATATTAATAGCAATCATAT TATTTCCCTTCAAAACAATTCATCCAG) (SEQ ID No. 6) had 37 of its 52 nucleotides complementary to the *gdm*Q sequence, so that the amplicons could anneal upon mixing.

Amplification of the 364 bp sequence of *gdmQ* were done by PCR with the primer pair *gdm*Q-*Eco*RI-2-F (CGGAATTCGTCTATCAATTCATCATCAA TG) (SEQ ID No. 7) and *gdm*Q-R (TGAAGGGAAATAATATGATTGCTAT TAATATTGTAGGTG) (SEQ ID No. 8) using *S. gallinarum* chromosomal DNA as template. Then the kanamycin resistance gene was amplified by PCR using the primers *kan-Cla*I-F (TTATCGATGCCGTATGTAAGGATTCAG) (SEQ ID No. 9) and *kan*-R (CCTACAATATTAATAGCAATCATATTATTTTCCTT CAAAACAATTCATCCAG) (SEQ ID No. 6) and plasmid pDG782 as a template.

Mixing the two PCR fragments, a new PCR was performed using the outer primers *gdm*Q-*EcoR*I-2-F (CGGAATTCGTCTATCAATTCATCATCAATG) (SEQ ID No. 7) and *kan-Cla*I-F (TTATCGATGCCGTATGTAAGGATTCAG) (SEQ ID No. 9). In order to efficiently perform overlap extension PCR, it was of fundamental importance that the melting temperature (T_{M}) of the overlapping sequence and the outer primers were the same. The PCR product (1510 bp), was digested with *Eco*RI and *Cla*I and ligated to *Eco*RI, *Acc*I digested pUC18NotI-P. The resulting plasmid was named pGV4 (Fig. 3A).

An *Escherichia coli-Staphylococcus* shuttle vector was constructed from plasmid pPSM1058 containing a temperature-sensitive (TS) staphylococcus-replicon, and the genes encoding resistance to ampicillin and chloramphenicol (Madsen et al., 2002). A ca.1.4 kb *Sac*I*-Nhe*I fragment containing the nuclease gene was excised and replaced by a *Sac*I-*Not*I-*Spe*I DNA fragment excised from pGEM5Zf(+) (Stratagene, La Jolla, CA) multiple cloning site. The resulting plasmid, pGV2 was used to construct the *gdm*P knock-out vector (Fig. 3B).

The knock-out vector pGV5 was assembled by cloning the approximately 2.2 kb *Not*I from pGV4, containing the internal *gdm*P fragment and the *kan*^{R}-*gdm*Q construct, into pGV2, the TS *Escherichia coli-Staphylococcus* shuttle vector. This knock-out vector was then transformed by electroporation into *Staphylococcus gallinarum* (Fig. 3B).

Transformation of *Staphylococcus gallinarum* required a high staphylococcal DNA concentration of more than 1µg/µl. Since pGV5 is a low copy number plasmid, its preparation in large enough quantities was achieved best by passing the plasmid through *Staphylococcus aureus* RN4220 as described elsewhere (Augustin & Götz, 1990). The resulting strain *Staphylococcus aureus* RN4220 (pGV5) was grown at 30°C in B-Medium (1% tryptone, 0.5% yeast extract 0.5% NaCl, 0.1% glucose, and 0.1% K₂HPO₄*3H₂O (pH 7.2)) supplemented with 20 µg/ml chloramphenicol. A three ml over night culture was used to inoculate a 25 ml culture (1:100) that was grown for 10 hours and used to inoculate a 1-L culture (1:100). This culture was allowed to grow into late stationary phase (16hours) before cells were harvested by centrifugation at 6,000 g, 4°C for 15 min. Plasmid was then isolated and purified with a Qiagen Plasmid purification kit (Qiagen, Hilden, Germany) following a modified protocol. The cell pellet was washed with 40 ml 0.15-mM EDTA-washing buffer, pH 8, then centrifuged at 5,000 g, 4°C for 10 minutes, the supernatant discarded, and the cell pellet was resuspended in 60 ml NaCl-buffer (50-mM Tris, 2.5-M NaCl, 50-mM EDTA, pH 7). 50 µl lysostaphin (10 mg/ml, AMBI, USA) were added to the suspension, which was then incubated at 37°C until viscosity increased (40 minutes). Thereafter, 60 ml lysis buffer (50-mM Tris, 300-mM EDTA, 0.5% Brij 58, 0.04% Na-deoxycholat, pH 8) at 4°C were added. The cell suspension was mixed and incubated on ice for 1 hour. Cell debris was removed by centrifugation with 19,000 g, 4°C for 30 min. The supernatant was filtered before 0.4 vol. of 50% PEG 6,000 were added. After mixing, the solution was incubated 2.5 hours on ice for DNA precipitation to proceed. The precipitated DNA was collected by centrifugation with 5,500 g, 4°C for 20 minutes, discarding the supernatant. The DNA pellet was resuspended in 4 ml buffer P1 (10-mM EDTA, 0.01% RNase, 50-mM Tris-HCl, pH8), and 4 ml buffer P2 (200-mM NaOH, 1% SDS) were added. After careful mixing the solution was incubated 5 minutes at room temperature. Thereafter, 4 ml buffer P3 (3-M potassium acetate, pH 5.5) at 4°C were added and the tubes immediately inverted 4-6 times. After 10 minutes incubation on ice followed by centrifugation with 15,000 g, 4°C for 30 minutes the cleared lysate was applied onto an anion exchange column (Qiagen Midi Plasmid Kit), which was then washed twice with QC washing buffer (1-M NaCl, 50-mM MOPS, pH 7, 15% isopropanol). The DNA was eluted with 2 ml buffer QF (1.25-M NaCl, 50-mM Tris-HCl, pH 8.5, 15% isopropanol). The DNA solution was precipitated by adding 0.7x vol. isopropanol vortexing, and centrifugation with 14,000 g, 4°C for 15 min. The DNA pellet was washed twice with 70% ethanol, then dried, and dissolved in 20 µl 1xTE, pH 8 (Sambrook et al. 1989). This isolation method yielded about 2 µg/µl of pGV5 that was used for electroporation of *Staphylococcus gallinarum.*

Electrocompetent cells of *Staphylococcus gallinarum* were obtained by preparing a ten ml culture B-Medium (1% tryptone, 0.5% yeast extract 0.5% NaCl, 0.1 % glucose, 0.1% K₂HPO₄*3H₂O, pH 7.2) incubated overnight, shaking with 250 rpm at 37°C. Five ml of this culture were used to inoculate 500 ml B-medium. The culture was grown until an OD₆₀₀ of between 0.45 and 0.5 was reached. The cells were centrifuged at RT, then washed with 500 ml washing buffer (0.75-M sucrose, 10-mM EDTA, pH 7.5). This washing step was repeated twice using 250 ml of washing buffer. The pellet was resuspended in a volume of washing buffer that corresponds in ml to the OD₆₀₀ value at which cells were harvested. Aliquots of 50 µl were distributed into cryo vials and stored at -80°C.

For electroporation, the cells were thawed on ice and incubated for 20 min with 4 µl plasmid DNA (ca. 4-8 µg). Then cells were transferred into an ice-cold 0.2 com cuvette, and electroporated with the BioRad Genepulser at 2.5 kV, 1000Ω and 1 µF. Resulting time constants were 0.6 ms. Then 1 ml SMMP70 medium (SMMP 70 consisted of 7.5 parts of 2x filter-sterilised SMM (1-M sucrose, 40-mM maleic acid, 40-mM MgCl₂, pH adjusted with NaOH to 6.8); 2 parts autoclaved Pennassay broth; and 0.5 parts of filter-sterilised 10% bovine serum albumin) was added, and the cells were incubated for 2 hours at 30°C, shaking with 225 rpm. Thereafter, cells were plated on selective B-agar plates, B-medium containing 1.2% agar, supplemented with 20 µg/ml chloramphenicol and incubated at 30°C. First colonies were visible after 24 h. Plates were totally incubated for 48 h.

The electroporation conditions balance ideally the conditions for DNA uptake by *Staphylococcus gallinarum* while preventing excessive cell death.

Following transformation and selection, gene replacement was induced in the S. *gallinarum* (pGV5) isolates grown at 30°C in B-medium under various selective conditions following the general outline described by Brückner (1997) (Fig. 4). In the first step, the transformed plasmid is forced to integrate site specific into the chromosome, conferring resistance to both chloramphenicol (Cm^{R}, plasmid marker) and kanamycin (Kan^{R}; *gdm*P replacement construct) at the non-permissible temperature. In a second step, colonies needed to be identified that have performed the 2^{nd} cross over and consequently have lost the vector part of pGV5 and the associated chloramphenicol resistance. Those clones were then analyzed for the presence of a chromosomally integrated copy of *kan*^{R} and on their ability to produce gallidermin and pre-gallidermin, respectively.

A first *S. gallinarum* (pGV5) culture was started inoculating 10 ml kanamycin-supplemented (25 µg/µl) B-medium with a single colony of a transformed cell. Culture was incubated shaking with 225 rpm at 30°C, a temperature that allowed pGV5 to replicate as free plasmid (permissive temperature), until late stationary phase (16h). With this culture, new cultures were inoculated (1:100) and incubated shaking with 225 rpm at 30°C into the late stationary phase (16h). Of these, 100 ml cultures in B-medium containing 7.5 µg/µl kanamycin (MIC value) were inoculated (1:100) and grown for 24 hours at the non-permissive temperature of 40°C. This step was repeated once, before diluting the cultures 1:100 into 100 ml B-medium containing no antibiotic. Growth at 40°C was extended for another 24 hours. The selection for plasmid integration resulted in 253 colonies with Cm^{R}Kan^{R} phenotype.

A 1: 10,000 dilution of the last culture was spread onto B-agar plates supplemented with 7.5 µg/µl kanamycin. After over night incubation at 40°C, colonies were replicated onto B-medium plates supplemented with either chloramphenicol (20µg/µl) or kananmycin (7.5 µg/µl). All colonies, which were found to be sensitive to chloramphenicol but resistant to kanamycin, were strains where double homologous recombination took place. Three colonies, designated *Staphyloccus gallinarum* Δ*gdm*P::*kan*, with Cm^{S} Kan^{R} phenotype were isolated and further analyzed.

The site of integration was analyzed by PCR using primers designed to generate characteristic amplicons for *Staphyloccus gallinarum* Δ*gdm*P::*kan* and the *Staphyloccus gallinarum* wild-type chromosomal DNA, which was isolated from 8 ml Luria Broth grown cultures (14 hours) using the Genomic tips (Qiagen, Hilden, Germany) with a modified protocol, omitting the proteinase K treatment for lysis, but including addition of 7 µl lysostaphin (10 mg/ml) in addition to lysozyme. Incubation at 37°C was extended to one hour. The second incubation was performed at 55°C instead of 50°C. In the final step, DNA was eluted with 3 ml hot (50°C) buffer QF (1.25-M NaCl, 50-mM Tris-HCl, pH 8.5, 15% isopropanol). DNA was removed with a glass rod and dissolved in 1xTE, pH 8. The PCR reactions were performed with 1:10 diluted DNA of which five microliter where used as DNA template. PCR reactions were performed with primer pairs PROOF-K (ACGAACTCCAATTCACTGTTCCTTG) (SEQ ID No. 10) and PROOF-P (GGTGAGGGGTGCTATATGAAGAAATT) (SEQ ID No. 11), and with PROOF-Q (CTTTGCACACCCTTAAATTATCTCTTAATC) (SEQ ID No. 12) and PROOF-P.

PCR reactions were analysed in a 0.8% agarose gel. Amplification with KP primer pair yielded a 1.14 kb fragment indicating the proper integration of the *kan* gene in *Staphyloccus gallinarum* Δ*gdm*P::*kan*; no KP primer product was identified in the wild-type. Also the QP primer pair resulted in the expected amplicon length of 2.5 kb and 2 kb for Staphyloccus *gallinarum* Δ*gdm*P::kan and the wild-type, respectively (Fig. 5 A/B).

Following the successful genetic examination *Staphyloccus gallinarum* Δ*gdm*P::*kan* culture supernatant was analyzed by HPLC for the presence of gallidermin and pre-gallidermin. Cultures were grown in YE4 medium (5% yeast extract (Ohly Cat, Deutsche Hefe Werke, Germany), 4.5% CaCl₂, and 0.5% maltose, pH 7.2). A three ml preculture was incubated shaking with 190 rpm for 24 hours at 37 °C and then used to inoculate (1:100) a 100 ml YE4 culture (in 250 ml Erlenmeyer flasks with one baffle) that was grown for 18 hours under the same conditions. Filtered culture supernatants were subjected to reversed-phase liquid chromatography (C18 Nucleosil column). The mobile phase was composed of 0.1% aqueous H₃PO₄ (v/v) (solvent A) and 100% acetonitrile (solvent B). Gallidermin and pre-gallidermin were separated using a linear gradient from 90% A/10% B to 45% A/55% B in 14 minutes at a flow rate of 1 ml/min. Regeneration of the column and equilibrium to start conditions was achieved in five more minutes. Analysis revealed that the *Staphyloccus gallinarum* wild-type supernatant contained gallidermin, calculated molecular mass of 2165.6 Da (peak at ca 9.7 minutes). Pre-gallidermins (peaks at ca. 9.1 and 9.5 minutes) was not detected (Figure 6A). In contrast, no gallidermin was detected in the supernatant of *Staphyloccus gallinarum* Δ*gdm*P::*kan.* Surprisingly, also the *gdm*A deduced sized pre-gallidermin was not detected. Instead only two truncated pre-gallidermin molecules were detected (Table 1; Fig. 6B). The main portion was identified as a 3408 Da pre-gallidermin, while a minor compound was associated to the 3621 Da precursor. All compounds were analysed by ESI-MS with a Water Q-TOF Ultima API in cationic mode (Fig. 6A/B). Apparently export of pre-gallidermin resulted in a partial cleavage of the leader peptide yielding these novel, so far not described molecules. Both of the truncated precursors were, however, properly processed into mature and active gallidermin (Table 1, Example 3).

It is possible that these truncated forms are produced during translocation through the membrane. Furthermore, this information may be used to alter the GdmA gene such as to shorten the native leader sequence or to modify the leader, with e.g. a His-tags, labels or other derivations to facilitate certain downstream processing steps, such as capture chromatography and a like.

**Table 1**

| ***Structure*** | ***Molecule*** | ***Mass expected [Da]*** | ***Mass detected [Da]*** |
|---|---|---|---|
| MEAVKEKNELFDLDVKVNAKESNDSGA EPR-Gallidermin (SEQ ID No. 3) | Expected sized pregallidermin | 6051.7 | Not detected |
| AKESNDSGAEPR-Gallidermin(SEQ ID No. 2) | Observed sized pregallidermins | 3408 | 3407.4 |
| VNAKESNDSGAEPR-Gallidermin(SEQ ID No. 1) | | 3621.3 | 3620.5 |
| Gallidermin | Mature gallidermin | 2165.6 | 2165.2 |

Further evidence for the lack of gallidermin production and the precursor accumulation in strain *Staphyloccus gallinarum* Δ*gdm*P::*kan* came from protein analysis of the culture supernatants by Tricine-SDS-PAGE, blotting and silver staining of the proteins. Tricine-SDS-PAGE (16% acrylamide) that allows separation of proteins from 1 to 100 kDa was done following the protocol described elsewhere (Schägger & von Jagow 1987) Samples, 15 µl of a 1:5 dilution of supernatants were adjusted to pH 8. Purified gallidermin (Alexis Corp) dissolved in water was used as standard. To allow for detection of very small peptides like gallidermin and pre-gallidermin, gels are transferred to a nitrocellulose membrane and then stained with silver staining (Kovarik et al. 1987). Blotting was done using a BIO RAD blotting apparatus. Prior to blotting, the transfer pads, filter pads and 0.45 µm nitrocellulose membranes (PROTRAN, Schleicher and Schuell) had to be soaked for at least 15 min in 4°C transfer buffer

(3 g Tris, 1.4 g glycine, 0.1% SDS, and 200 ml MeOH, ad 1 L). Blotting sandwich was prepared, and blotting was performed in 4°C transfer buffer at 64 mA for 40 minutes. A protocol described elsewhere was used for silver staining the membrane (Kovarik et al. 1987). As shown in Fig. 7 both gallidermin and the truncated 3408 Da form of pre-gallidermin could be detected by this technique. While the wild-type *Staphylococcus gallinarum* only produced gallidermin (2165.6 Da) in detectable quantities, the GdmP deficient mutant only produced the larger precursor.

To further substantiate this, supernatants from both wild-type and mutant strain was subjected to a bioassay following the protocol of Kies and coworker (Kies, et al., 2003). Briefly, *Kocuria rhizophila* (formerly *Micrococcus luteus*) was grown in LB-medium at 30°C for 24 hours. The culture was diluted to an OD₆₀₀ of one. Then 0.3 ml of this dilution were mixed into 150 ml cooled molten B-agar, to which 0.3 ml 50% glucose were added. Plates containing 10 ml medium were prepared. 20µl supernatant of test cultures were pipetted onto 6 mm sterile paper disks. A negative control, 20 µl of medium YE4, was applied as well. Dried filters were placed onto the *K. rhizophila* containing plates, which were then incubated at 30°C for 24 hours. The bioassay (Fig. 8A) confirmed previous data. Only supernatant from wild-type Staphylococcus *gallinarum* had an inhibitory effect on the sensitive microorganism. *Staphylococcus gallinarum* Δ*gdm*P::*kan* showed no inhibitory effect at all.

These results show that the GdmP protease mutant strain *Staphylococcus gallinarum* Δ*gdm*P::*kan* indeed lacked the expression of a functional extracellular protease that is required to proteolytically process pre-gallidermin precursor molecules into mature and active gallidermin. Moreover, it was shown that strain *Staphylococcus gallinarum* Δ*gdm*P::*kan* accumulated' novel truncated pre-gallidermin in the culture supernatant.

While the generation of a GdmP deletion strain is the most critical step during the strain development program, other genetic modifications to increase the production of pre-gallidermin are obvious to those skilled in the art and include increasing the gene dosage by adding an additional copy of the *gdm*P deleted gallidermin cluster, removal of the no longer required genes *lan*I and *lan*EFG encoding the immunity functions, changing promoter strength and regulation including and alike, known to those skilled in the art can be employed to further improve the strain's productivity.

### Example 2

### Fermentative production of pre-gallidermin

Gallidermin is produced by *Staphylococcus gallinarum* Tü3928. Several protocols have been described in the recent literature that detailed the production and yields of gallidermin and its partial purification (Fiedler et al., 1988; Kellner et al., 1988; Kempf et al., 2001; EP0342486). These protocols include the direct adsorption of gallidermin from the fermentation broth with Amberlite XAD-1180. The water washed resin was eluted with acidified methanol, and the gallidermin was recovered by drying the eluate *in vacuo* yielding some 2.7 g crude material from a 20-L fermentation. The crude gallidermin was further purified by ion exchange chromatography on Amberlite IRC-50 resin followed by a desalting step again on XAD-1180 yielding about 1g of the crude antibiotic. Final purification was achieved on a reversed-phase chromatography with a 10C18 column, reducing the yield to 100 mg purified gallidermin obtained from a 30-L fermentation. Due to the toxicity of gallidermin to the producing organism, all the efforts to increase yields focussed in fermentation regimes that require online product removal or repeated partial harvests (Kempf et al., 2001).

We found a process using an engineered strain of *Staphylococcus gallinarum* that is deficient in the final step of proteolytic gallidermin activation (example 1). Said strain only makes the non-toxic and non-active gallidermin precursor molecules, exported into the culture supernatant. Said pre-gallidermin was then partially purified for its biocatalytic activation (example 4).

Production of these truncated pre-forms of gallidermin allows for all fermentation regimes known to those skilled in the art, which include high, cell density, batch-, fed-batch or continuous cultivation with either complete or repeated partial harvests; all with or without in situ product removal strategies.

To evaluate and prove that the gallidermin precursor molecules are indeed non-toxic to the producing organism, dilutions of a partially purified pre-gallidermin stock solution were prepared of which 20 µl aliquots were applied to filter discs that were then placed onto GM plate freshly inoculated with wild type S. *gallinarum.* As shown in Fig. 8B, no inhibition of growth was observed for the concentrations examined, up to 8 g/l precursor per litre, clearly demonstrating the non-toxicity of the gallidermin precursor molecules.

The following cultivations show the potential of this approach to reach production titres well above the current limit of 300-400 mg/l, at which the gallidermin toxicity prevents further improvement of fermentation titres. Fig. 9 shows an example for a fed batch fermentation of *Staphyloccus gallinarum* Δ*gdm*P::*kan* using the following set-up. Initially, we modified the YE medium replacing the CaCl₂ by 2% NaCl. This measure eliminated the otherwise observed calcium precipitation without affecting growth or gallidermin production. A 3 ml seed culture in modified YE medium is inoculated with a single colony of a freshly grown B-medium agar plate. After over night incubation at 37°C shaking at 225 rpm, an OD₆₀₀ of some 4.7 is reached and the seed culture (same medium) is used to inoculate the pre-culture (1:100). After ca. 10 hours incubation at 37° C shaking with 225 rpm, the exponentially growing pre-culture is used to inoculate a bioreactor (1:60) containing the modified YE medium. Cultivation parameters are set and controlled as follows: temperature: 37°C; pH: 6.5, controlled by the addition of NaOH and H₂SO₄; air flow: 1 vvm, up to 3 vvm; dissolved oxygen tension: >30%, controlled by air flow, stirrer speed and feed rate. Antifoam, Bayer Industrol DF 204, BASF Corporation, Gurnee, IL, was to added 0.01% and supplemented as required. A maltose feed (5%) was initiated after DOT started to raise (ca. 10 hours). Under the chosen conditions, the fermentation was completed in 24 hours. HPLC analysis of the filtered broth and conservative manual peak integration resulted in a yield of about 0.8 g/l pre-gallidermin, which is equivalent to approximately 0.5 g/l gallidermin. On a molar basis titres obtained for the precursor molecules are well above those obtained for gallidermin (ca. 150-200 mg/l) using the wild-type strains and processes resulting in the production of mature gallidermin in the fermentation.
The analytical procedure for quantification and determination of the purity of pre-gallidermin and gallidermin, respectively, were as follows.
A La Chrom system (Merck, Germany) equipped with an L-7100 pump, an L-7200 autosampler with a 100 µl injection loop; an L-7455 diode array detector (DAD) and an L-7490 refractive index detector (RI) was used. Data collection was done with a Merck-Hitachi model D-7000 chromatography station software. A Prontosil Eurobond C18 5.0 pm, (125 X 4.6 mm I.D.) with guard column (10 X 4.6 mm I.D.) of the same material (Bischoff, Leonberg, Germany) was used. The injection volume was 10 µl; column temperature was maintained at 25°C. The mobile phase was made up from 0.1% H₃PO₄ (mobile phase A) and acetonitrile (Mobile phase B). Separation was achieved at a flow rate of 1 ml/min and a linear gradient that is detailed in the table below:

| Time [min] | Mobile phase |
|---|---|
| 0 | 90%A |
| 1 | 90%A |
| 14 | 45%A |
| 15 | 45%A |
| 15.1 | 90%A |
| 19.5 | 90%A |

Further improvements in the fermentation protocol will evidently result in higher titers. In particular, since gallidermin precursor production is growth associated and that extending growth by feeding an utilisable source of carbon such as glycerol with or without supplementation with utilisable sources of nitrogen, sulphur, and or other micro nutrients such as vitamins or selected amino acids such as prolin, cystine, serine or lysins or any other amino acid or mixes thereof, will improve the fermentation outcome. Also replacement of the yeast extract by e.g soya meal or protein or cotton seed flower that can contain amino acids and micro elements more amenable for good growth of *S*. *gallinarum,* will further improve growth.

The fermentation regime and data are provided as an example only and does not preclude further improvement in productivity by employing techniques commonly used by those skilled in the art to optimise the fermentation regime including media composition, feed composition and feed rates, as well as other commonly controlled parameter that will alter the growth kinetic and production dynamics of a microbial cultivation.

### Example 3

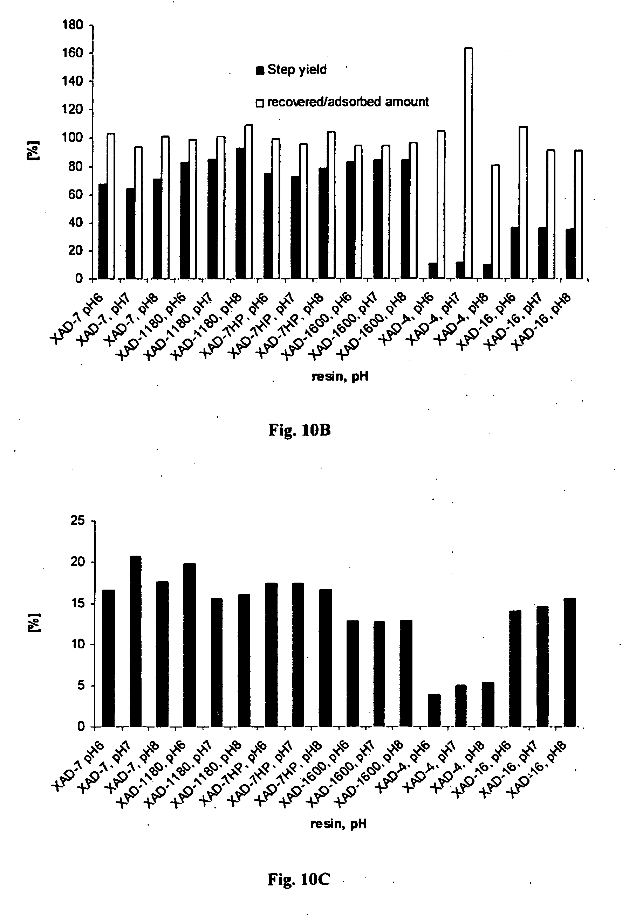

Following the fermentation, the initial steps of the isolation of the gallidermin precursor molecules involve the removal of the biomass using filtration technologies or centrifugation. For demonstration purposes, centrifugation with 10,000 x g for 10 minutes was employed. The clear, cell-free supernatant containing the gallidermin precursor molecules was than applied to a hydrophobic interaction chromatography using commercially available resins such as Amberlite XAD1600, XAD1180, XAD16, XAD7, XAD7HP and XAD-4 Amberlite resins and all chemicals were provided from Fluka (Buchs, Switzerland), Rohm & Haas (Philadelphia, PA, U.S.A.) or Roth (Reinach, Switzerland) unless mentioned otherwise. It was critical for that resins were washed prior use. Packed into a column (20x1.5 cm I.D.) resins were washed with 8 bed volumes methanol. The alcohol was then displaced with 8 bed volumes of double distilled water prior to adsorption. Adsorption was tested at pH 6, 7 and 8 by adjusting the pH of the cleared with 5 mM H₂SO₄ to pH 6, 7, or 8 and prior to loading of the aforementioned resins. 1800 µl cell free fermentation supernatant and 200 µl 1-M Na-phosphate buffer were added into a 2ml Eppendorf tube. Eighty mg (dry weight basis) washed resin were added to the solution and incubated at 30°C, shaking at 220 rpm. After 90 min incubation, liquid phase and resin phase were separated by short centrifugation; pre-gallidermin concentration in the supernatant was determined by HPLC. Desorption was carried out with an elution buffer composed of (9:1, v/v) methanol / 5mM H₂SO₄ (pH 4). Elution buffer, 600 µl, was added to the resin and incubated at 30°C, shaking with 220 rpm. After 30 min incubation liquid phase and resin phase were separated by short centrifugation and the pre-gallidermin concentration in the supernatant was determined. After removal of the liquid phase, a second 600 µl elution buffer were added to the resin phase and the same procedure was repeated. The results of these screens are summarized in Figures 10A to 10C. Resin capacities and elution efficiency are summarized in Figure 10A, step yield and the ratio between recovered and adsorbed material for the different resins and pH conditions are summarized in Figure 10B. Figure 10C summarized the HPLC purity of the eluted product. Taking together all results, best overall results were obtained with XAD1180 at pH 6.5; XAD1180 selectivity was lightly enhanced at pH6, but at pH 6.5, fermentation broth may be loaded without further pH adjustment. Performing the adsorption / desorption in a column rather then in a batch process is expected to increase the step yield. These conditions are distinctly different from those reported in EP0342486 were adsorption was done at the end of the cultivation at pH 8.5, conditions that were not suitable for isolating of the performs of gallidermin.
The methanol from the eluate was evaporated in vacuo at up to 40°C to obtain a methanol free preparation of pregallidermin for further analysis.

### Example 4

### Proteolytic in vitro activation of pre-gallidermin into pallidermin

The serine-type protease GdmP cleaves the pre-gallidermin molecule recognizing the amino acid sequence Ala-Glu-Pro-Arg-↓-Ile-gallidermin of the N-terminal leader peptide, and cleaving off the leader peptide between arginie and isoleucine as indicated by the arrow. The protease cleavage site has been described earlier (Ottenwälder et al., 1995; Bierbaum et al., 1996; Furmanek et al, 1999). Nevertheless, reports on the in-vitro proteolytic digestion of type-A lantibiotics are controversial. Gallidermin and epidermin, both are digested by trypsin under standard conditions to yield several inactive fragments (Allgaier et al., 1986; Kellner et al., 1988) that were used for structural analysis. This result is not surprising, as both molecules possess several potential trypsin cleavage sites. Hence it is not surprising, that an activation of pre-gallidermin using proteases other then GdmP or EpiP has never been reported. Other lantibiotics, such as nisin that do not possess additional sites for proteolysis may be derived by in-vitro proteolysis under standard conditions (US2004/0009550 (A1)).

Trypsin has an aspartate residue (189) at the bottom of the pocket of the active site, and this Asp forms a salt bridge with the positively charged group at the end of the substrate Lysine and Arginine side chains, on which this enzyme acts. The two relevant sites in gallidermin are the C-terminal arginine residue of the leader peptide and a lysine at position 13. This lysine, however, is located next to a 2,3-dihydrobutyrin (Dhb) residue hat was thought to possibly be protected under when tryptic digestion is done under carefully selected non-standard conditions. In order to identify proteases and conditions that would only cleave off specifically the N-terminal leader peptide, pre-gallidermin isolated from cultivations (see example 2 and 3) was incubated in the presence of several serine proteases, including clostripain (endonuclease ArgC, Roche Diagnostics), papain, bromelain (*Ananas comosus*) and trypsin using' choosing conditions that was not obvious to those skilled in the art.

Endonuclease ArgC (Roche Diagnostics), a proteinase isolated from *Clostridium histolyticum* cleaving peptide bonds C-terminal from arginine as found in pre-gallidermin, was examined for converting gallidermin precursor molecules into gallidermin. A crude pre-gallidermin powder obtained by foam separation from a fermentation of *Staphylococcus gallinarum* Tü3928 that contained mostly pre-gallidermin with only traces of gallidermin. Resuspended in 100 µl incubation buffer (100-mM Tris-HCl, 10-mM CaCl₂, pH 7.6) Of these 85 µl, 10 µl activation buffer (50-mM DTT, 5-mM EDTA) and 5 µl endoproteinase ArgC were mixed. Following incubation for 12 hours at 37°C, a sample was taken and the gallidermin/pre-gallidermin ratio was determined by HPLC and compared to the initial ratio. After 12 h incubation with ArgC, the gallidermin/pre-gallidermin ratio increased from 1:9.8 to 1:1.54 and the amount of gallidermin increased as expected (Fig. 11A), demonstrating the successful conversion. Bioassay results (not shown) confirmed an increase in antibiotic activity after ArgC treatment. As this protease is expensive and not very efficient in processing the gallidermin molecules, alternative proteases were evaluated.

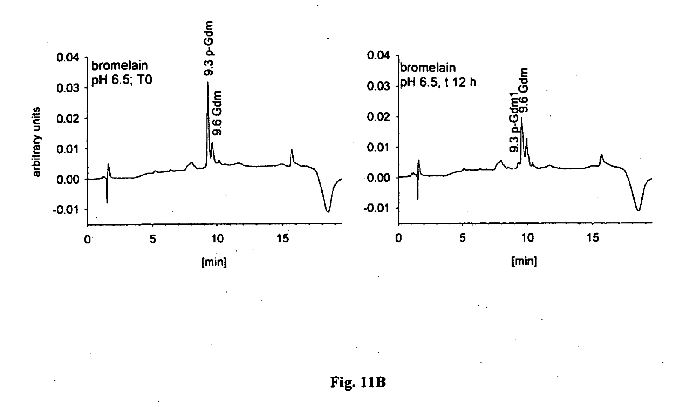

Papain (American Laboratories Inc, 2000 USP (IU) ca 30%) and stem bromelain (American Laboratories Inc, 600 GDU ca 25%) were evaluated using an aqueous solution of partially purified pre-gallidermin (250 mg/l) was adjusted to pH 6.5 and incubated with Bromelain 600 (0.05 g/l; American Laboratories) at room temperature (- 20°C) for 12h. (Fig. 11B). Most of the pre-gallidermin was converted into gallidermin, a significant portion, however, resulted in side products.

In contrast, papain Papain (160 mg/l) in 15 mM potassium phosphate buffer did not convert the gallidermin precursor (∼ 500 mg/l) molecules (not shown).

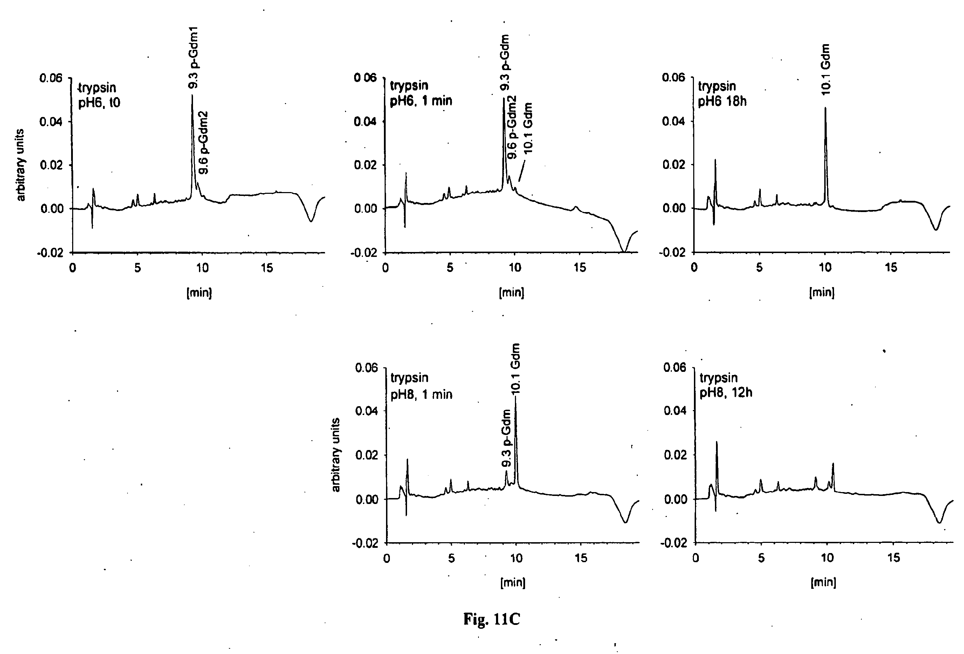

Surprisingly trypsin that previously had been reported to digest and to inactivate gallidermin was the most suitable enzyme, albeit under conditions not commonly used. Gallidermin precursor molecules isolated as outlined in example 2 were subjected to proteolysis with trypsin. Figure 11C depicts the results of this tryptic digests under acidic (pH 6) and alkaline (pH 8) conditions using 250 mg/l gallidermin precursor molecules with 50 mg/l trypsin (America Laboratories Inc, 1:200) incubated at room temperature (RT) for up to 20 hours. While at pH 6 the complete conversion of the precursor molecules into mature and active gallidermin was achieved within hours (Fig. 11C), alkaline conditions which are known as typical reaction conditions for trypsin, resulted in a much more rapid conversion but with the possibility that gallidermin is degraded upon prolonged exposure. Improved conversion of partially purified pre-gallidermin, 90% by HPCL area, was obtained at pH 6.5, 20°C using 1.1g/l pre-gallidermin and 0.2 g/l trypsin. Conversion was completed within minutes (Fig. 11D).

The selective protease digestion conditions, as defined by the selective processing of pre-lantibiotics, can be further optimized in a variety of ways. This can include, for example, low protease to lantibiotic ratio, decreased temperature, altered pH, etc. The various relevant parameters can be altered independently or in combination.

Of all proteases examined, only trypsin appeared to be suitable for a commercial and industrialised process. In order to demonstrate that the tryptic digest indeed produced active and mature gallidermin, samples of trypsin-treated pre-gallidermin were used in a bioassay, alongside with gallidermin isolated from *Staphylococcus gallinarum* Tü3928 cultivations. ESI-MS analysis (Water Q-TOT Ultima API in cationic mode) of the tryptic digest (Fig. 12A) resulted in single compound with a mass of 2165.2 Da corresponding to that of gallidermin isolated from the wild-type strain and within the analytical precision to the calculated mass of 2165.5 Da. As shown in Fig. 12B in-situ activated gallidermin exhibited the same bioactivity then gallidermin isolated from the wild-type strain for the concentrations indicated. We have disclosed the feasibility to selectively cleave pre-gallidermins into mature gallidermin using serine-type proteases and trypsin in particular. While we have employed soluble enzyme preparations, alternatives, such as immobilized enzyme preparations, could be used instead. This would facilitate the repetitive use of such enzymes and avoid possible contaminations of the final product by trypsin, even though, trypsin is generally regarded as safe (GRAS).

### Example 5

After identification of trypsin as a suitable protease, the conditions were analyzed in more details. In particular the use of an industrially and pharmaceutically amenable enzyme, gamma-irradiated trypsin (porcine 1:250, powder, SAFC Biosciences, USA) was compared to the trypsin from American Laboratories Inc. used in example 4. Methanol free and pre-gallidermin solutions containing 20, 40, and 60% methanol were evaluated under the following conditions: trypsin was used at 200 mg/l, pre-gallidermin concentration was ca. 700 mg/l in 25 mM Na-phosphate buffer pH 6; reaction volume was 1000 µl. The activities were calculated during the first 7 minutes of the reaction and the reaction yields are based on the maximum gallidermin obtained during the reaction. The results obtained with the gamma-irradiated trypsin are summarized in the Table below and in Figures 13 A trough 13D.

| Results of the tryptic conversion of pre-gallidermin to gallidermin using gamma-irradiated trypsin. | | | | | |
|---|---|---|---|---|---|
| | Formula | 0% Methanol | 20% Methanol | 40% Methanol | 60% Methanol |
| Activity [µmol min¹] | ([Gdm]ₜ₁-[Gdm]ₜ₀)/(t1-t0) | 0.019 | 0.021 | 0.016 | 0.007 |
| Specific actifity [µmol s⁻¹ mg_{trypsin}g¹] | ([Gdm]ₜ₁-[Gdm]ₜ₀)/((t1-t0)*0.2 mg_{trypsin}ml⁻¹) | 0.388 | 0.427 | 0.310 | 0.148 |
| Reaction yield [%] | _{Max}[Gdm]/[P/gdm]ₜ₀*100 | 99.6 | 106 | 105 | 107 |

Interestingly there was no difference in catalytic activity and gallidermin yield in the presence of 0 or 20% methanol in the reaction (Figure 13 A & B); the presence of 40% methanol extended the reaction time needed to 100 % conversion from ca. 0.8 hours to 1.2 hours; and even in the presence of 60 % methanol is pre-gallidermin completely converted in ca. 3 hours. Conversions were stochiometrical and no loss of pre-gallidermin or formation of side products was detected (Figure 14).

### Example 6

### Purification of proteolytically activated gallidermin

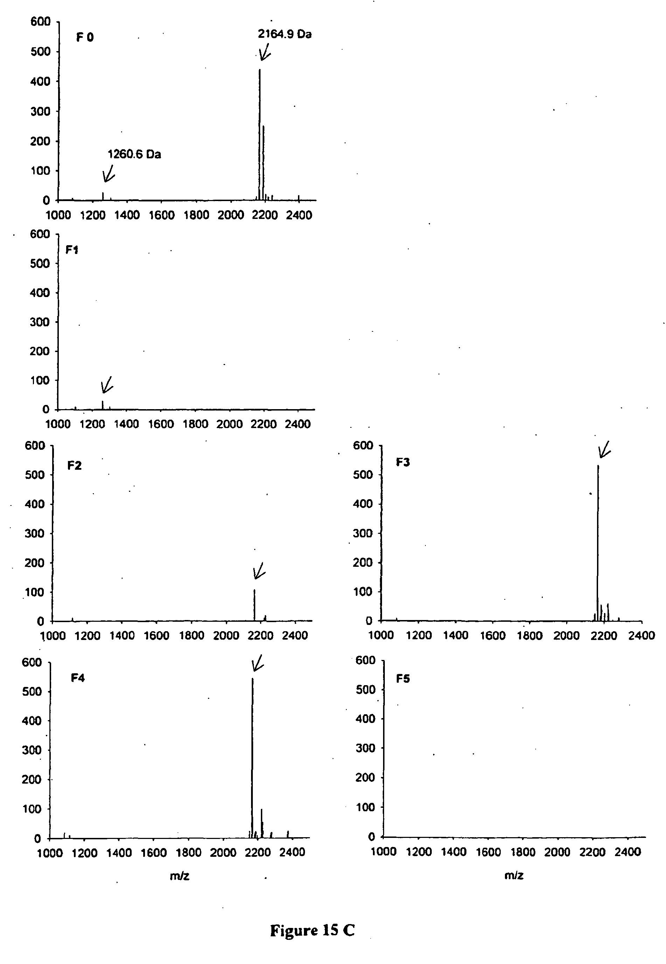

This example describes one possible way to isolate maturated gallidermin after its proteolytic activation. The initial tryptic digest was performed with 3 g/l pre-gallidermin in solution. After tryptic digest was completed (Figure 15 A and B) 0.7 ml to the reaction were separated by preparative HPLC on a ProntoSIL 120-10-C18 column. The mobile phase for the isocratic separation with 10 ml/min flow rate was made of acetonitrile: 0.1% TFA, 27.5:72.5 (v/v). Five fractions were collected as indicated in Figure 15 C and analyzed by mass spectrometry. As indicated by the presence or absence of the 2164.9 Da gallidermin peak (2165.5 Da theoretical mass) in the fractions, it was determined that fraction 1 and 5 contained no gallidermin. While some gallidermin was detected in fraction 2, most was found in fractions 3 and 4. The sample directly taken from the digest and fraction F1 from the preparative HPLC both presented a mass peak of 1260.6 Da that is likely to represent the predominant pre-gallidermin leader sequence plus the hydroxyl introduced during cleavage. This mass is not detected in the later fractions. Hence we conclude that we could separate the native gallidermin form it's cleaved off leader peptide. The fractions containing the gallidermin peak were dried evaporated in vacuo at up to 40°C to obtain a solvent free preparation of gallidermin for bioassay analysis. The bioassay confirmed that active gallidermin was present in fractions 2-4.

Further purification steps employing hydrophobic interaction chromatography with e.g. Amberlite XAD1180 or equivalent, ion exchange chromatography and gelfiltration to yield pure and dried gallidermin have been published elsewhere (Fiedler et al., 1988).

The invention is not limited to the embodiments hereinbefore described, with reference to the accompanying drawings, which may be varied in construction and detail.

### References

Allgaier, H., G. Jung, R.G. Werner, U. Schneider and H. Zähner, 1986. Epidermin: Sequencing of a heterodetic tetracyclic 21-peptide amide antibiotic. Eur. J. Biochem. 160(1):9-22.
Augustin, J., and F. Götz, 1990. Transformation of Staphylococcus epidermidis and other staphylococcal species with plasmid DNA by electroporation. FEMS Microbiol Lett 54:203-7.
Bierbaum, G., F. Götz, A. Peschel, T. Kupke, M. van der Kamp, and H. G. Sahl. 1996. The biosynthesis of the lantibiotics epidermin, gallidermin, Pep5 and epilancin K7. Antonie Van Leeuwenhoek International Journal of Gen Mol Microbiol 69:119-127.
Bonelli, R. R., T. Schneider, H. G. Sahl, and I., Wiedemann, 2006. Insights into in vivo activities of lantibiotics from gallidermin and epidermin mode-of-action studies. Antimicrob. Agents Chemother. 50(4): 1449-57.
Brückner, R. 1997. Gene replacement in Staphylococcus carnosus and Staphylococcus xylosus. FEMS Microbiol Lett 151:1-8.
Breukink E. and de Kruijff B., 2006. Lipid II as a target for antibiotics. Nat. Rev. Drug Discov. 5(4): 321-32.
de Vuyst, L. and E. Vandamme, 1993. Nisin a lantibiotic produced by Lactococcus lactis supsp. lactis: properties, biosynthesis, fermentation and applications. In: de Vuyst, L. and E. Vandamme (eds.) "Bactiocins of lactic acid bacteria: Microbiology, genetics and applications". Elsevier Applied Biotech. Series, pp151-221.
Fiedler, H-P., T. Hörner and H. Decker, 1988. Purification of the hydrophilic antibiotics epidermin, gallidermin, and nikkomycin Z by preparative reversed-phase HPLC. Chromatographia 26:215-220.
Furmanek, B., T. Kaczorowski, R. Bugalski, K. Bielawski, J. Bohdanowicz, A. J. Podhajska, and J. Bogdanowicz. 1999. Identification, characterization and purification of the lantibiotic staphylococcin T, a natural gallidermin variant. J Appl Microbiol 87:856-66.
Götz, F. and G. Jung, 2001. Biosynthesis of the lantibiotics epidermin and gallidermin. In: Braun, V. and F. Götz (ed.) "Microbial fundamentals of biotechnology". WILEY-VCH Verlag GmbH, Weinheim, pp:52-92.
Guerot-Fleury, A.-M., N. Shazand, N. Frandsen and P. Stragier. 1995. Antibiotic resistance cassettes for Bacillus subtilis. Gene 167:335-336.
Herrero, M., V. de Lorenzo and K. N. Timmis, 1990. Transposon vectors containing non-antibiotic resistance selection markers for cloning and stable chromosomal insertion of foreign genes in gram-negative bacteria. J. Bacteriol. 172:6557-67.
Hille, M. 2002. Untersuchungen zur Biosynthese der Lantibiotika Gallidermin und Epidermin. Dissertation University of Tubingen, Germany.
Hille, M., S. Kies, F. Götz and A. Peschel, 2001. Dual role of GdmH in producer immunity and secretion of the staphylococcal lantibiotics gallidermin and epidermin. Appl. Environ. Microbiol. 67:1380-1383.
Jack, R.W., G. Bierbaum and H.-G. Sahl (eds.) 1998. Lantibiotics and related peptides, Springer-Verlag Berlin, pp.224.
Kellner, R., G. Jung, T. Hörner, H. Zähner, N. Schnell, K.-D. Entian and F. Götz, 1988. Gallidermin: a new lanthionine-containing polypeptide antibiotic. Eur. J. Biochem. 177:53-59.
Kempf, M., U. Theobald and H.-P. Fiedler, 2001. The antibiotic gallidermin - evolution of a production process. In: Van Broekhoven, A., F. Shapiro and J. Anne (eds.) "Novel frontiers in the production of compounds for biomedical use". Vol. 1, Kluwer Academic Publishers, Dordrecht, pp 35-55.
Kies, S., C. Vuong, M. Hille, A. Peschel, C. Meyer, F. Götz and M. Otto, 2003. Control of antimicrobial peptide synthesis by the agr quorum sensing system in Staphylococcus epidermidis: activity of the lantibiotic epidermin is regulated at the level of precursor processing. Peptides 24:329-338.
Kovarik, A., K. Hlubinova, A. Vrbenska and J. Prachar, 1987. An improved colloidal silver staining method of protein blots on nitrocellulose membranes. Folia Biol (Praha) 33:253-7.
Madsen, S. M., H. C. Beck, P. Ravn, A. Vrang, A. M. Hansen, and H. Israelsen. 2002. Cloning and inactivation of a branched-chain-amino-acid aminotransferase gene from Staphylococcus carnosus and characterization of the enzyme. Appl Environ Microbiol 68:4007-14.
McAuliffe, O., R. P. Ross and C. Hill, 2001. Lantibiotics: structure, biosynthesis and mode of action. FEMS Microbiol. Rev. 25:285-308.
Ottenwälder, B., T. Kupke, S. Brecht, V. Gnau, J. Metzger, G. Jung, and F. Götz. 1995. Isolation and characterization of genetically engineered gallidermin and epidermin analogs. Appl Environ Microbiol 61:3894-903.
Sahl, H. G. and G. Bierbaum, 1998. Lantibiotics: biosynthesis and biological activities of uniquely modified peptides from gram-positive bacteria. Annu. Rev. Microbiol. 52:41-79.
Sambrook, J., E.F. Fritsch and T. Maniatis, 1989. Molecular Cloning, a laboratory manual, Second Edition, C old Spring Harbor Laboratory Press, NY.
Schägger, H. and G. von Jagow (1987). "Tricine sodium dodecyl-sulfate polyacrylamide-gel electrophoresis for the separation of proteins in the range from 1-Kda to 100-Kda." Anal. Biochem. 166: 368-379.

## Claims

1. A process for the preparation of an inactive pre-form of gallidermin comprising the steps of :-
culturing a biologically pure organism capable of producing gallidermin wherein the gallidermin specific serine protease (GdmP) gene of the biologically pure organism is inactivated by the insertion of a constitutive promoter cassette; and
isolating the inactive pre-form of gallidermin from the cultivation.

2. A process as claimed in claim 1 wherein the constitutive promoter cassette is a kanamycin resistance promoter cassette.

3. A process as claimed in any of claims 1 to 2 wherein the organism is cultivated in an aqueous cultivation medium containing assimilable sources of carbon, nitrogen and inorganic substances until substantial growth and metabolic activity is detectable.

4. A process as claimed in any of claims 1 to 3 wherein the inactive pre-form of the gallidermin is isolated by separation and subsequently purified.

5. A process as claimed in claim 4 wherein the separation comprises centrifugation or filtration.

6. A process as claimed in claim 4 or 5 wherein the purification comprises chromatography, such as hydrophobic interaction chromatography.

7. A process as claimed in claim 6 wherein the chromatography is carried out under neutral to acidic conditions.

8. A process as claimed in claim 6 or 7 wherein the inactive pre-form of gallidermin is purified by hydrophobic interaction chromatography in methanolic solvent mixtures.

9. A process as claimed in any of claims 6 to 8 wherein the eluate from the chromatographic process contains the inactive pre-form of gallidermin.

10. A process as claimed in any preceding claim wherein the inactive pre-form of gallidermin is biocatalytically activated to yield a mature and active form of gallidermin.

11. A process as claimed in claim 10 wherein the inactive pre-form of gallidermin is biocatalytically activated with a protease selected from any one or more of ArgC, bromelain and trypsin.

12. A' process as claimed in claim 11 wherein the inactive pre-form of gallidermin is biocatalytically activated under acidic pH conditions.

13. A process as claimed in claim 12 wherein the inactive pre-form of gallidermin is biocatalytically activated at pH 6.5.

14. A process as claimed in claim 12 wherein the inactive pre-form of gallidermin is biocatalytically activated at pH 6.0.

15. A process as claimed in any of claims 10 to 14 further comprising the step of purifying the active form of gallidermin

16. A process as claimed in claim 15 wherein the active form of gallidermin is purified by one or more chromatographic techniques selected from: ion exchange chromatography, reverse phase chromatography, gel filtration chromatography, and preparative HPLC.

17. A process as claimed in any of claims 1 to 16 wherein gallidermin and the isolated pre-form of gallidermin comprises a truncated amino acid sequence of VNAKESNDSGAEPR (SEQ ID No. 1).

18. A process as claimed in any of claims 1 to 17 wherein gallidermin and the isolated pre-form of gallidermin comprises a truncated amino acid sequence AKESNDSGAEPR (SEQ ID No. 2) or any other N-terminally truncated pre-forms.

19. A process as claimed in any of claims 1 to 18 wherein gallidermin and the isolated preform comprises a geneticallymodified GdmA gene.

20. A genetically modified strain of *Staphlylococcus gallinarum* wherein the gallidermin specific serine protease, (GdmP) gene is inactivated by the insertion of a constitutive promoter cassette.

21. A modified strain of *Staphlylococcus gallinarum, Staphylococcus gallinarum* Δ*gdm*P::*kan* deposited with the Deutsche Sammlung von Microorganismen having a depository number of DSM 17239.

## Patentansprüche

1. Verfahren zur Herstellung einer inaktiven Vorform von Gallidermin, das die folgenden Schritte umfasst:
Kultivieren eines biologisch reinen Organismus, der Gallidermin produzieren kann, worin das Gallidermin-spezifische Serinprotease- (GdmP) Gen des biologisch reinen Organismus durch die Insertion einer konstitutiven Promotorkassette inaktiviert ist; und
Isolieren der inaktiven Vorform von Gallidermin aus der Kultur.

2. Verfahren nach Anspruch 1, worin die konstitutive Promotorkassette eine Kanamycin-Resistenz-Promotorkassette ist.

3. Verfahren nach einem der Ansprüche 1 bis 2, worin der Organismus in einem wässrigen Kulturmedium, das assimilierbare Quellen für Kohlenstoff, Stickstoff und anorganische Substanzen enthält, kultiviert wird bis ein erhebliches Wachstum und Stoffwechselaktivität nachweisbar ist.

4. Verfahren nach einem der Ansprüche 1 bis 3, worin die inaktive Vorform des Gallidermins durch Abtrennung isoliert und anschließend aufgereinigt wird.

5. Verfahren nach Anspruch 4, worin die Abtrennung Zentrifugieren oder Filtrieren umfasst.

6. Verfahren nach Anspruch 4 oder 5, worin die Aufreinigung Chromatographie umfasst, wie beispielsweise hydrophobe Interaktionschromatographie.

7. Verfahren nach Anspruch 6, worin die Chromatographie unter neutralen bis sauren Bedingungen durchgeführt wird.

8. Verfahren nach Anspruch 6 oder 7, worin die inaktive Vorform von Gallidermin durch hydrophobe Interaktionschromatographie in methanolischen Lösungsmittelmischungen aufgereinigt wird.

9. Verfahren nach einem der Ansprüche 6 bis 8, worin das Eluat aus dem chromatographischen Verfahren die inaktive Vorform von Gallidermin enthält.

10. Verfahren nach einem vorstehenden Anspruch, worin die inaktive Vorform von Gallidermin biokatalytisch aktiviert wird, um eine reife und aktive Form von Gallidermin zu ergeben.

11. Verfahren nach Anspruch 10, worin die inaktive Vorform von Gallidermin mit einer Protease, die aus einer oder mehreren von ArgC, Bromelain und Trypsin ausgewählt ist, biokatalytisch aktiviert wird.

12. Verfahren nach Anspruch 11, worin die inaktive Vorform von Gallidermin unter Bedingungen von saurem pH biokatalytisch aktiviert wird.

13. Verfahren nach Anspruch 12, worin die inaktive Vorform von Gallidermin bei pH 6,5 biokatalytisch aktiviert wird.

14. Verfahren nach Anspruch 12, worin die inaktive Vorform von Gallidermin bei pH 6,0 biokatalytisch aktiviert wird.

15. Verfahren nach einem der Ansprüche 10 bis 14, das weiter den Schritt des Aufreinigens der aktiven Form von Gallidermin umfasst.

16. Verfahren nach Anspruch 15, worin die aktive Form von Gallidermin durch eine oder mehrere chromatographische Techniken aufgereinigt wird, die aus Folgenden ausgewählt sind: Ionenaustausch-Chromatographie, Umkehrphasen-Chromatographie, Gelfiltrations-Chromatographie und präparativer HPLC.

17. Verfahren nach einem der Ansprüche 1 bis 16, worin Gallidermin und die isolierte Vorform von Gallidermin eine gekürzte Aminosäuresequenz von VNAKESNDSGAEPR (SEQ.-ID-Nr. 1) umfassen.

18. Verfahren nach einem der Ansprüche 1 bis 17, worin Gallidermin und die isolierte Vorform von Gallidermin eine gekürzte Aminosäuresequenz von AKESNDSGAEPR (SEQ.-ID-Nr. 2) oder jedwede anderen N-terminal gekürzten Vorformen umfassen.

19. Verfahren nach einem der Ansprüche 1 bis 18, worin Gallidermin und die isolierte Vorform ein genetisch modifiziertes GdmA-Gen umfassen.

20. Genetisch modifizierter Stamm von *Staphylococcus gallinarum,* worin das Gallidermin-spezifische Serinprotease- (GdmP) Gen durch die Insertion einer konstitutiven Promotorkassette inaktiviert ist.

21. Modifizierter Stamm von *Staphylococcus gallinarum, Staphylococcus gallinarum* Δ*gdm*P::*kan,* der bei der Deutschen Sammlung von Mikroorganismen mit einer Hinterlegungs-Nummer von DSM 17239 hinterlegt ist.

## Revendications

1. Procédé de préparation d'une préforme inactive de la gallidermine, comprenant les étapes consistant à :
cultiver un organisme biologiquement pur capable de produire la gallidermine, le gène de la protéase à sérine spécifique de la gallidermine (GdmP) de l'organisme biologiquement pur étant inactivé par l'insertion d'une cassette contenant un promoteur constitutif ; et
isoler la préforme inactive de la gallidermine de la culture.

2. Procédé selon la revendication 1, dans lequel la cassette contenant un promoteur constitutif est une cassette de résistance à la kanamycine contenant un promoteur.

3. Procédé selon l'une quelconque des revendications 1 à 2, dans lequel l'organisme est cultivé dans un milieu de culture aqueux contenant des sources assimilables de carbone, d'azote et de substances inorganiques jusqu'à ce qu'une croissance et une activité métabolique importantes soient décelables.

4. Procédé selon l'une quelconque des revendications 1 à 3, dans lequel la préforme inactive de la gallidermine est isolée par séparation et est ensuite purifiée.

5. Procédé selon la revendication 4, dans laquelle la séparation comprend une centrifugation ou une filtration.

6. Procédé selon la revendication 4 ou 5, dans lequel la purification comprend une technique de chromatographie, comme la chromatographie d'interactions hydrophobes.

7. Procédé selon la revendication 6, dans lequel la chromatographie est effectuée dans des conditions neutres à acides.

8. Procédé selon la revendication 6 ou 7, dans lequel la préforme inactive de la gallidermine est purifiée par chromatographie d'interactions hydrophobes dans des mélanges de solvants méthanoliques.

9. Procédé selon l'une quelconque des revendications 6 à 8, dans lequel l'éluat de la chromatographie contient la préforme inactive de la gallidermine.

10. Procédé selon l'une quelconque des revendications précédentes, dans lequel la préforme inactive de la gallidermine est activée par biocatalyse pour produire une forme mature et active de la gallidermine.

11. Procédé selon la revendication 10, dans lequel la préforme inactive de la gallidermine est activée par biocatalyse avec une protéase sélectionnée parmi l'ArgC, la bromélaïne et/ou la trypsine.

12. Procédé selon la revendication 11, dans lequel la préforme inactive de la gallidermine est activée par biocatalyse dans des conditions de pH acide.

13. Procédé selon la revendication 12, dans lequel la préforme inactive de la gallidermine est activée par biocatalyse à pH 6,5.

14. Procédé selon la revendication 12, dans lequel la préforme inactive de la gallidermine est activée par biocatalyse à pH 6,0.

15. Procédé selon l'une quelconque des revendications 10 à 14, qui comprend également l'étape consistant à purifier la forme active de la gallidermine.

16. Procédé selon la revendication 15, dans lequel la forme active de la gallidermine est purifiée par une ou plusieurs techniques de chromatographie sélectionnées parmi les suivantes : chromatographie d'échange d'ions, chromatographie en phase inverse, chromatographie par filtration sur gel et CLHP préparatoire.

17. Procédé selon l'une quelconque des revendications 1 à 16, dans lequel la gallidermine et la préforme isolée de la gallidermine comprennent une séquence d'acides aminés tronquée de la séquence VNAKESNDSGAEPR (SÉQ ID N° : 1).

18. Procédé selon l'une quelconque des revendications 1 à 17, dans lequel la gallidermine et la préforme isolée de la gallidermine comprennent une séquence d'acides aminés tronquée AKESNDSGAEPR (SÉQ ID N° : 2) ou toute autre préforme tronquée à l'extrémité N-terminale.

19. Procédé selon l'une quelconque des revendications 1 à 18, dans lequel la gallidermine et la préforme isolée comprennent un gène GdmA génétiquement modifié.

20. Souche génétiquement modifiée de *Staphylococcus gallinarum,* dans laquelle le gène de la protéase à sérine spécifique de la gallidermine (GdmP) est inactivé par l'insertion d'une cassette contenant un promoteur constitutif.

21. Souche modifiée de *Staphylococcus gallinarum, Staphylococcus gallinarum* Δ*gdm*P::*kan*, déposée auprès du *Deutsche Sammlung von Microorganismen* sous le numéro d'accession DSM 17239.
